# FASCICULE DE BREVET EUROPEEN

(11) **EP 0 839 815 B1**
(45) Date de publication et mention de la délivrance du brevet: **01.03.2000**
(21) Numéro de dépôt: 97402316.0
(22) Date de dépôt: 02.10.1997
(51) Int. Cl.: C07F 7/08, C07F 7/21, A61K 7/42

(54) **Utilisation de composés siliciés en teinture des fibres kératiniques humaines; nouveaux composés et compositions les contenant**
Verwendung von siliciumhaltigen Verbindungen zum Färben von Menschlichen Keratinfasern; neue Verbindungen und Zusammensetzungen, die diese enthalten.
Use of silicon compounds for dyeing human keratinic fibres; compounds and compositions

(30) Priorité: 30.10.1996 FR 9613267
(43) Date de publication de la demande: 06.05.1998
(73) Titulaire: L'OREAL, 75008 Paris (FR)
(72) Inventeur: Leduc, Madeleine, 75011 Paris (FR); Richard, Hervé, 93420 Villepinte (FR); Lagrange, Alain, 77700 Coupvray (FR)
(74) Mandataire: Miszputen, Laurent

(56) Documents cités:
- GB-A- 882 063
- US-A- 2 931 693
- US-A- 4 038 293
- CHEMICAL ABSTRACTS, vol. 75, no. 24, 13 décembre 1971 Columbus, Ohio, US; abstract no. 141953y, GORBACHEVA, I.N. ET AL.: "synthesis and study of dyeing properties of silicon containing dyes" XP002032545 & NAUCH.-ISSLED. TR. MOSK. TEKST. INST., 1969, pages 298-304,
- CHEMICAL ABSTRACTS, vol. 123, no. 12, 18 septembre 1995 Columbus, Ohio, US; abstract no. 146685g, MAKSIMOVA, G.V. ET AL.: "interaction of epoxy-containing organosilicon compounds with anthraquinone dyes" XP002032546 & VYSOKOMOL. SOEDIN., SER A SER. B, vol. 37, no. 3, 1995, pages 558-560,

## Description

L'invention concerne l'utilisation de composés siliciés, linéaires ou cycliques, comportant au moins un groupement chromophore de type quinonique ou azoïque à titre de colorants directs dans une composition tinctoriale destinée à la teinture des fibres kératiniques humaines et en particulier des cheveux; elle concerne également certains de ces composés siliciés à titre de composés nouveaux.

On a déjà décrit et proposé des colorants siliciés pour colorer des fibres synthétiques; de tels colorants sont décrits dans les brevets belges N° 875 160 - 875 182 - 875 230, américain N° 2 925 313, allemand N° 2 918 685 ou dans la demande européenne N° 455 595.
On a également décrit et proposé des colorants siliciés pour colorer des résines de silicone, voir à ce propos les brevets US 3 888 891, 3 981 859 et 4 038 293.
On connait aussi dans l'état de la technique des colorants siliciés utilisables dans la fabrication de polymères photosensibles, voir la demande de brevet allemand N° 2 040 831.
Pour la plupart d'entre eux, ces colorants siliciés sont réactifs en raison des groupements hydrolysables qu'ils portent dans leur structure et n'ont jamais été proposés pour leur utilisation en cosmétique.
Par ailleurs, certains colorants polysiloxaniques non réactifs mais physiquement et chimiquement inertes et de haut poids moléculaire ont été préparés dans le brevet américain N° 4 381 260, et proposés en raison de leur stabilité, pour des utilisations telles que la coloration des textiles naturels, des matières plastiques, ou pour colorer les aliments, les préparations pharmaceutiques et cosmétiques.

Or, dans le domaine de la teinture capillaire, on recherche des colorants directs, c'est-à-dire des colorants qui, sans l'apport d'agent oxydant, sont capables de modifier par eux-mêmes la nuance naturelle des cheveux, de façon temporaire. Dans cette application, les colorants doivent satisfaire à un certain nombre de critères; ils doivent en outre être le plus inoffensif possible c'est-à-dire présenter une innocuité acceptable, et engendrer des colorations suffisamment résistantes, en particulier à la lumière, aux lavages, à la transpiration et aux intempéries.

Parmi les colorants directs classiquement utilisés en teinture capillaire, on connait des colorants quinoniques tels que ceux de type anthraquinone, benzoquinone, naphtoquinone et des colorants azoïques. Tous ces colorants ne sont pas complètement inoffensifs, c'est pourquoi en cosmétique capillaire, on recherche des molécules colorantes de ce type toujours plus performantes en terme d'innocuité.

Or, après d'importantes recherches menées sur la question, la Demanderesse vient maintenant de découvrir que des composés siliciés qui comportent au moins un groupement chromophore de type quinonique ou azoïque, et dont certains sont nouveaux permettent de teindre sans oxydant les fibres kératiniques humaines telles que les cheveux, avec la propriété avantageuse de présenter une excellente innocuité.
En outre, les teintures capillaires obtenues à l'aide de ces composés sont plus résistantes que celles réalisées avec des composés non siliciés de type quinonique ou azoïque de l'art antérieur.

Cette découverte est à la base de la présente invention.

La présente invention a ainsi pour objet l'utilisation à titre de colorants directs dans une composition tinctoriale destinée à la teinture des fibres kératiniques humaines et en particulier des cheveux, de composés siliciés, linéaires ou cycliques, comportant au moins un groupement chromophore de type quinonique ou azoïque, caractérisés par le fait qu'ils répondent à l'une des formules (1), (2) ou (3) suivantes : 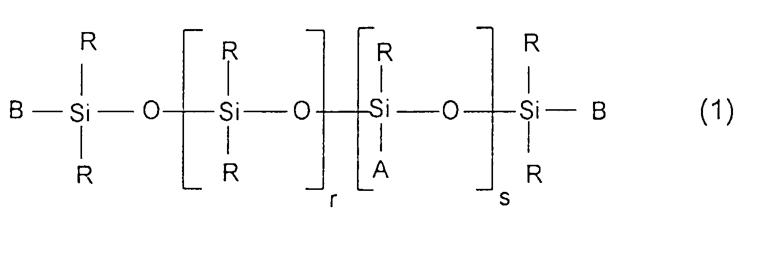 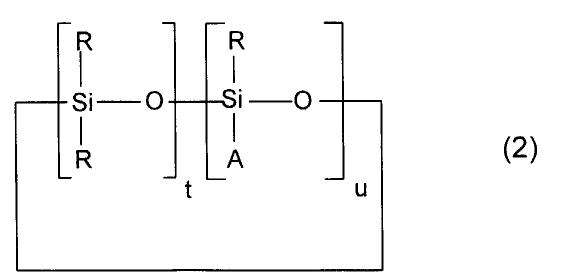 formules (1) ou (2) dans lesquelles :
- **R**, identiques ou différents, sont choisis parmi les radicaux alkyles en C₁-C₁₀, phényle et trifluoro-3,3,3 propyle, au moins 80% en nombre des radicaux R étant méthyle,
- **B, identiques ou différents, sont choisis parmi les radicaux R ci-dessus,**
- **un des deux radicaux B peut désigner le radical A défini ci-après,**
- **r** est un nombre entier compris entre 0 et 50 inclusivement, et **s** est un nombre entier compris entre 0 et 20 inclusivement, avec la condition que si s est nul **alors un des radicaux B désigne A,**
- **u** est un nombre entier compris entre 1 et 6 inclusivement, et
- **t** est un nombre entier compris entre 0 et 10 inclusivement, étant entendu que t + u est égal ou supérieur à 3, 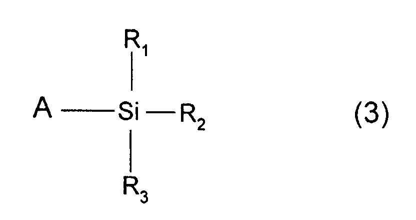 formule (3) dans laquelle:
   - **R**_{**1**}**, R**_{**2**}**, R**_{**3**}**,** identiques ou différents, sont choisis parmi les radicaux alkyles et alcényles en C₁-C₈, linéaires ou ramifiés, saturés ou insaturés,
   formules (1), (2) et (3) dans lesquelles le symbole **A** désigne un radical identique ou différent de formules (4a) (4b) (4c) (4d) suivantes : 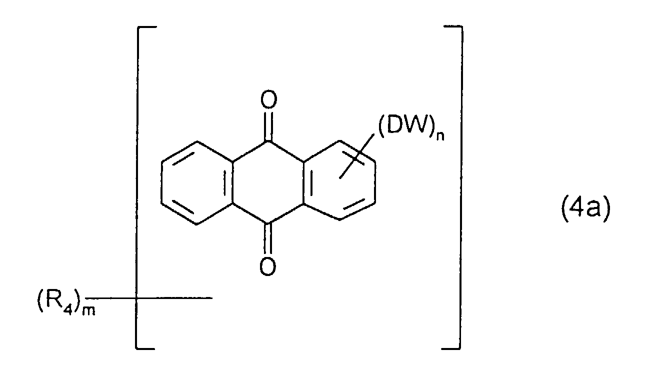 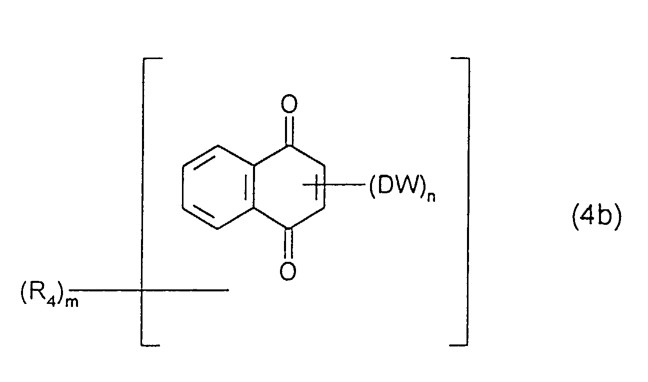 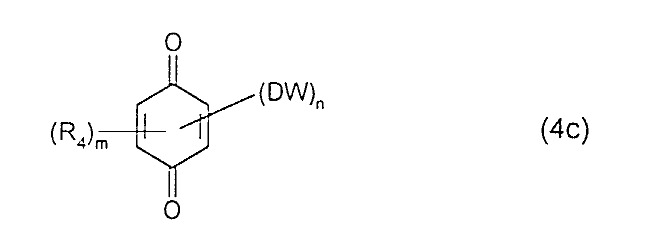 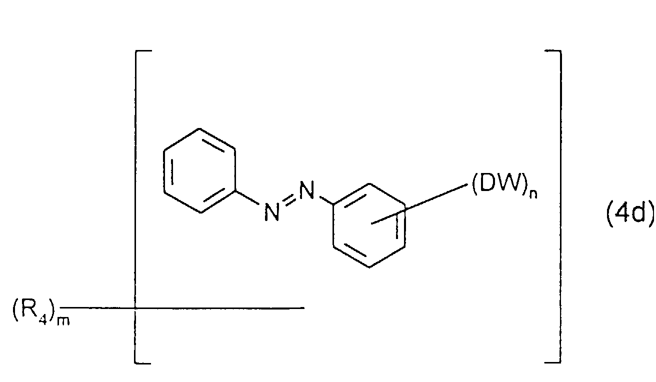 formules (4a) (4b) (4c) (4d) dans lesquelles :
- **R**_{**4**}, identiques ou différents, représentent un radical alkyl, linéaire ou ramifié en C₁-C₆, un radical OH, alcoxy en C₁-C₄, hydroxyalkyl(C₁-C₄), COOH, CONH₂, CN, SO₃H, halogène, NO₂, NR₅R₆ dans lequel **R**_{**5**} et **R**_{**6**}, identiques ou différents, désignent un atome d'hydrogène, un radical alkyl en C₁-C₈, ou hydroxyalkyl(C₁-C₄), ou aminoalkyl(C₁-C₄), ou forment ensemble avec l'atome d'azote auquel ils sont attachés un hétérocycle à 5 ou 6 chaînons éventuellement interrompu par un atome d'oxygène ou de soufre,
- **m** est un nombre entier compris entre 0 et 2 inclusivement,
- **n** est un nombre entier égal à 1 ou 2,
- **D** est est un radical -SO₂NH-, -CONH-, -O-, ou un radical -NR₇- dans lequel R₇ est H ou CH₃,
- **W** est un radical divalent de formule (5): 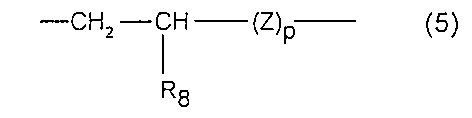 ou de formule (6):

   - HC = CH - (Z)ₚ- (6)

   formules (5) et (6) dans lesquelles :
- **R**_{**8**} désigne un atome d'hydrogène, un radical hydroxyle, un radical alkyl en C₁-C₈, linéaire ou ramifié, saturé ou insaturé,
- **Z** est un radical alkylène en C₁-C₆, linéaire ou ramifié éventuellement substitué par un radical OH ou un radical alcoxy en C₂-C₈ linéaire ou ramifié, saturé ou insaturé,
- **p** est un nombre entier égal à 0 ou 1.

Dans les formules (1), (2) ou (3) ci-dessus, A représente donc le groupement quinonique (anthraquinone, naphtoquinone, benzoquinone) ou azoique qui, après fixation sur les chaînes siliciées de départ confère aux composés de formules (1), (2) et (3) des propriétés colorantes.

Les radicaux alkyle peuvent être choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R et B préférés selon l'invention sont les radicaux méthyle, éthyle, propyle, n-butyle, n-octyle et éthyl-2 hexyle. Encore plus préférentiellement, les radicaux R et B sont tous des radicaux méthyle.

Parmi les composés siliciés linéaires ou cycliques utilisables selon la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement méthyle,
- B est alkyle et encore plus préférentiellement méthyle,
- r est compris entre 0 et 3 inclusivement et est plus préférentiellement nul,
- s est compris entre 1 et 3 inclusivement, plus préférentiellement égal à 1,
- t + u est compris entre 3 et 5 inclusivement,
- R₁, R₂, R₃ sont alkyle et encore plus préférentiellement méthyle,
- R₈ est hydrogène, Z est méthylène et p est égal à 1.

Selon l'invention, les composés plus particulièrement préférés sont les suivants :
- 4-(4-diméthylaminophênylazo)-N-[3-[1,3,3,3-tétraméthyl-1-[(triméthylsilyl)-oxy]-disiloxanyl]-propyl)-benzènesulfonamide [composé (7)] : 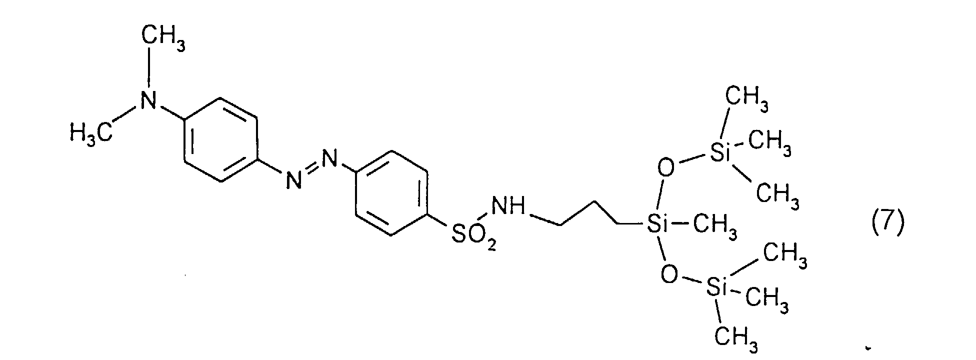
- 4-(4-diméthylaminophénylazo)-N-[3-[1,3,3,3-tétraméthyl-1-[(triméthylsilyl)-oxy]-disiloxanyl]-propyl]-benzamide [composé (8)] : 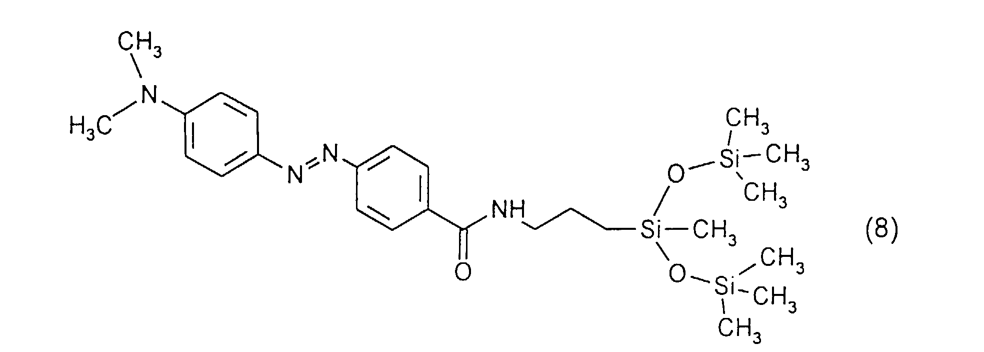
- 2-(4-méthoxy-2-nitrophénylazo)-5-(3-triméthylsilanyl-propoxy)-phénol [composé (9)]: 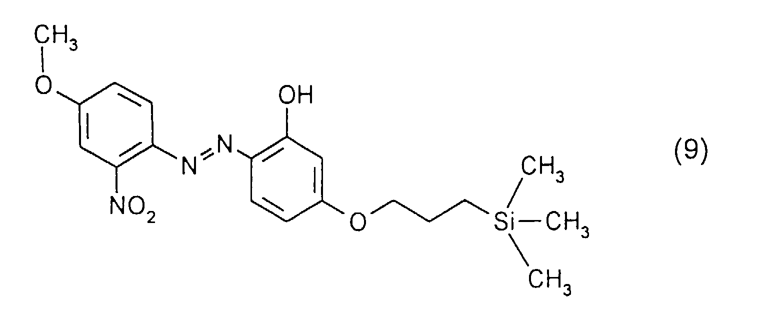
- 2,5-bis-[3-[1,3,3,3-tétraméthyl-1-[(triméthylsilyl)-oxy]-disiloxanyl]-propylamino]-benzoquinone [composé (10)]: 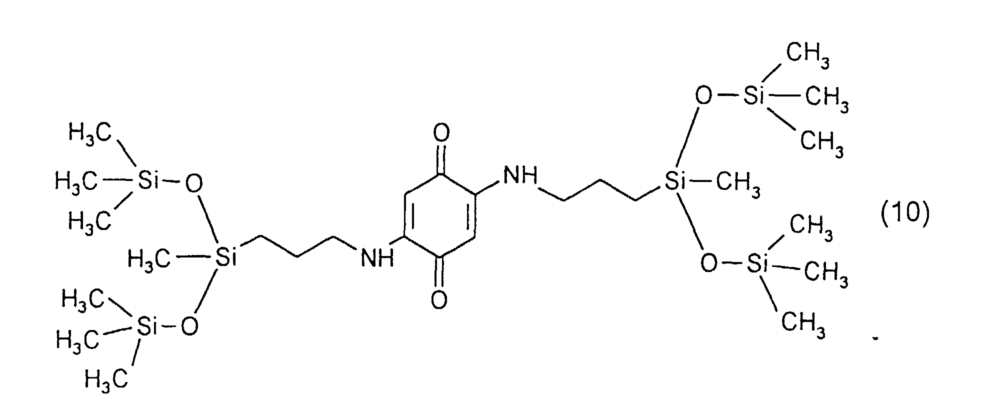
- 2-chloro-3-[3-[1,3,3,3-tétraméthyl-1-[(triméthylsilyl)-oxy]-disiloxanyl]-propylamino]-[1,4]-naphtoquinone [composé (11)]: 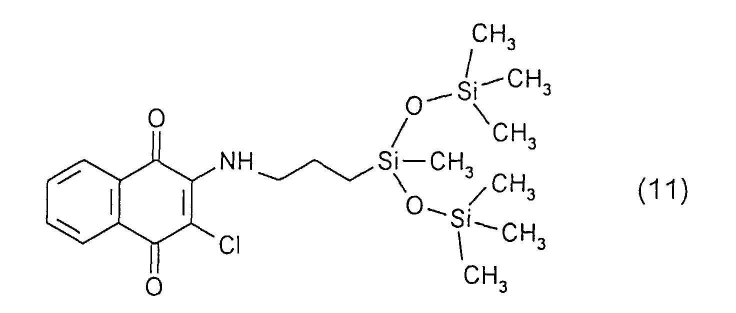
- 2-[3-[1,3,3,3-tétraméthyl-1-[(triméthylsilyl)-oxy]-disiloxanyl]-propylamino]-[1,4]-naphtoquinone [composé (12)]: 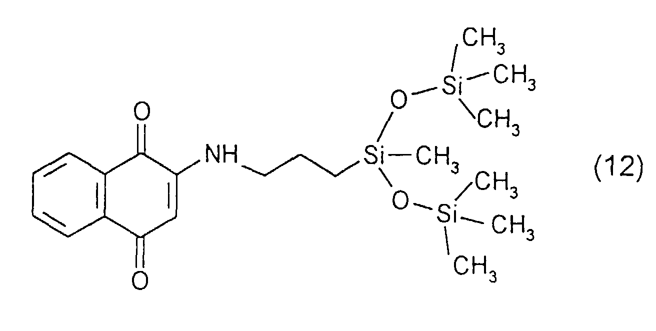
- 1-hydroxy-4-[3-[1,3,3,3-tétraméthyl-1-[(triméthylsilyl)-oxy]-disiloxanyl]-propylamino]-anthraquinone [composé (13)]: 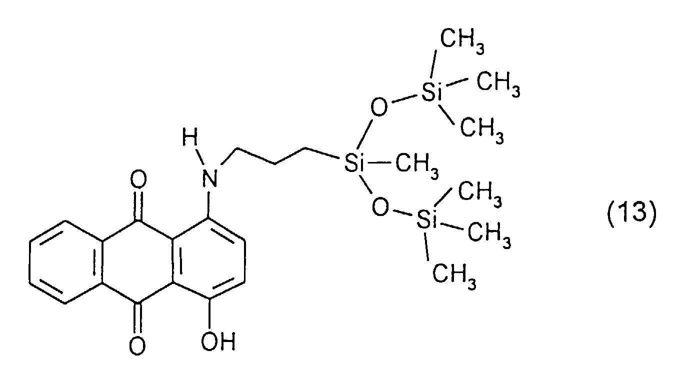
- 1,4-di-[3-[1,3,3,3-tétraméthyl-1-[(triméthylsilyl)-oxy]-disiloxanyl]-propylamino]-anthraquinone [composé (14)]:

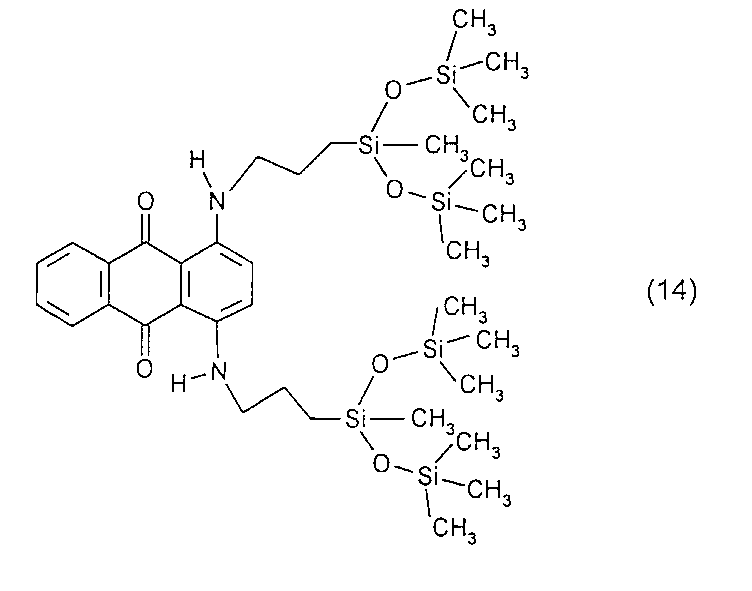

Pour préparer les colorants siliciés de formules (1), (2) et (3), on peut procéder classiquement (voie 1), en mettant en oeuvre une réaction d'hydrosilylation, à savoir : 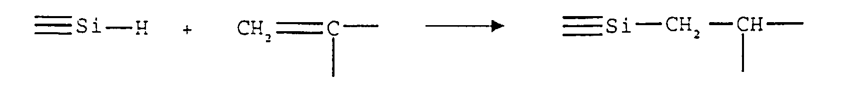 à partir du composé silicié correspondant, dans lequel, par exemple, tous les radicaux A sont des atomes d'hydrogène. Ce composé silicié de départ est dénommé par la suite dérivé à SiH ; les groupes SiH peuvent être présents dans la chaîne et/ou aux extrémités de la chaîne siliciée. Ces dérivés à SiH sont des produits bien connus dans l'industrie des silicones et sont généralement disponibles dans le commerce. Ils sont par exemple décrits dans les brevets américains N° 3 220 972, 3 697 473 et 4 340 709.
Ce dérivé à SiH peut donc être représenté,
- soit par la formule (15) suivante : 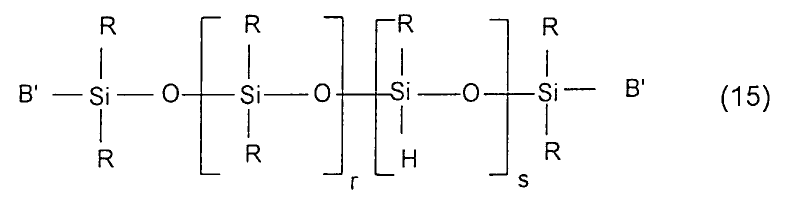 dans laquelle R, r et s ont la signification donnée ci-dessus pour la formule (1) et les radicaux B', identiques ou différents, sont choisis parmi les radicaux R et un atome d'hydrogène,
- soit par la formule (16) suivante : 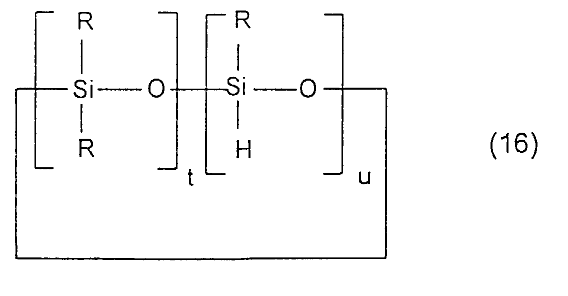 dans laquelle R, t et u ont la signification donnée ci-dessus pour la formule (2).
- ou par la formule (17) suivante : 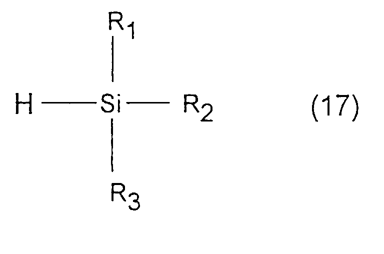 dans laquelle R₁, R₂, R₃ ont la signification donnée ci-dessus pour la formule (3).

Sur ce dérivé à SiH de formule (15), (16) ou (17), on effectue donc une réaction d'hydrosilylation classique, en présence d'une quantité catalytiquement efficace d'un catalyseur au platine, sur un composé organique de formules (4a), (4b), (4c), (4d), dans lesquelles le symbole W est remplacé par le symbole W' qui désigne un radical monovalent de formules (18) ou (19) suivantes :

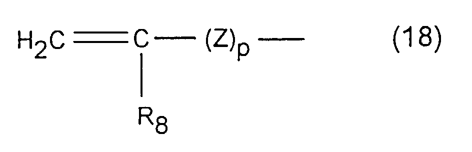

HC≡C-(Z)ₚ- (19)

formules (18) et (19) dans lesquelles, Z, R₈ et p ont les mêmes significations qu'aux formules (5) et (6) ci-avant définies.

Une autre voie de synthèse possible (voie 2) convenant à la préparation des colorants siliciés de formules (1), (2) et (3) consiste à faire réagir un composé silicié aminé correspondant aux formules (15), (16) ou (17) ci-avant définies, mais dans lesquelles l'atome d'hydrogène a été remplacé par un radical monovalent de formule : -D-CH₂-CHR₈-(Z)ₚ-NH₂, avec pour D, les mêmes significations qu'aux formules (4a), (4b), (4c), 4d) ci-avant définies et pour R₈ et p, les mêmes significations qu'aux formules (5), (6), (18) ou (19) ci-avant définies, sur un composé de formules (20a), (20b), (20c) ou (20d) suivantes : 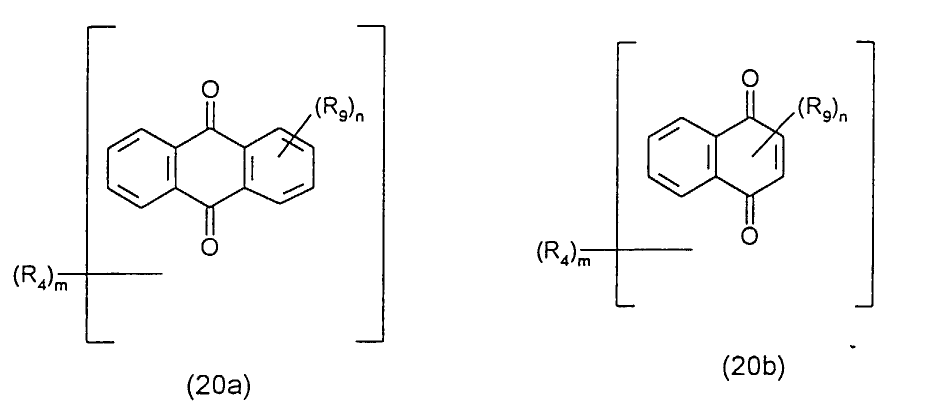 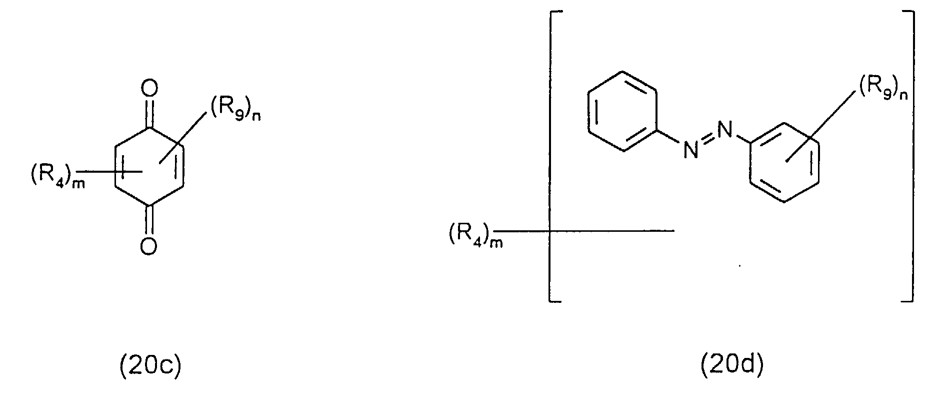 formules (20a), (20b), (20c) ou (20d) dans lesquelles :
- R₄, m et n ont les mêmes significations que dans les formules (4a), (4b), (4c), (4d) ci-avant définies,
- **R**_{**9**} désignant un atome d'hydrogène, un atome d'halogène ou un radical hydroxyle.

Parmi les composés de formule (1) de l'invention, certains sont connus en tant que tels, notamment ceux décrits dans les brevets US N° 4 381 260 et SU 1 712 374.
D'autres sont nouveaux et constituent un autre objet de la présente invention.

La présente invention a ainsi pour objet des composés nouveaux de formule (1) ci-après définie, et de formules (2) définie précédemment.

**Lesdits composés nouveaux de formule (1) sont ceux pour lesquels les radicaux B désignent le radical CH**_{**3**}**, s est égal à 1, et r désigne un nombre entier compris entre 0 et 10 inclusivement.**

Lesdits composés de formule (3) sont nouveaux à l'exception de ceux pour lesquels A est un groupe de formule (4d), R₈ désigne l'hydrogène, R₁, R₂ et R₃ désignent des groupes alkyle et D un groupe NR₇ qui sont décrits dans les brevets GB 882 063 et US 2 931 693.

L'invention a également pour objet une composition tinctoriale pour kératiniques humaines et notamment les cheveux, et plus particulièrement une composition de teinture directe pour cheveux, comprenant dans un milieu approprié pour la teinture, une quantité efficace d'au moins un composé de formule (1), (2), ou (3) définie ci-avant.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Les composés de formule (1), (2), ou (3) sont généralement présents dans des proportions comprises entre environ 0,01 et 10%, de préférence entre environ 0,1 et 5% en poids, par rapport au poids total de la composition de teinture.

Les composés de formule (1), (2), ou (3) peuvent également être incorporés dans des compositions tinctoriales pour la coloration d'oxydation qui contiennent des bases d'oxydation et éventuellement des coupleurs, pour enrichir en reflets les nuances obtenues avec les colorants d'oxydation.

Le milieu approprié pour la teinture est alors de préférence un milieu constitué par de l'eau et/ou des solvants organiques acceptables sur le plan cosmétique, et plus particulièrement, des alcools (l'alcool éthylique, l'alcool isopropylique, l'alcool benzylique, et l'alcool phényléthylique), des glycols ou éthers de glycol (le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, les éthers monométhylique, monoéthylique et monobutylique d'éthylèneglycol), dans des concentrations comprises entre environ 0,5 et 20% et, de préférence, entre environ 2 et 10% en poids par rapport au poids total de la composition.

Ladite composition tinctoriale peut contenir en outre tout autre adjuvant utilisé habituellement en teinture de fibres kératiniques humaines, et par exemple, des agents tensio-actifs bien connus de l'état de la technique et de type anionique, cationique, non-ionique, amphotère, zwittérionique ou leurs mélanges, des agents épaississants, des agents anti-oxydants, des parfums, des agents séquestrants, des agents dispersants, des agents de conditionnement, des agents conservateurs, des agents opacifiants, etc...
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition de teinture selon l'invention peut être formulée à pH acide, neutre ou alcalin, le pH pouvant varier par exemple de 4 à 11 et de préférence de 5 à 10, et pouvant être ajusté au moyen d'agents d'alcalinisation ou d'agents d'acidification antérieurement bien connus.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 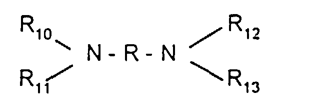 dans laquelle R est un reste propylène éventuellement substitué par un groupement hydroxyle ou un radical alkyle en C₁-C₄ ; R₁₀, R₁₁, R₁₂ et R₁₃, identiques ou différents, représentent un atome d'hydrogène, un radical alkyle en C₁-C₄ ou hydroxyalkyle en C₁-C₄.
Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition appliquée sur les fibres peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Un autre objet de l'invention porte sur un procédé de teinture des fibres kératiniques humaines, et notamment des cheveux, par coloration directe, consistant à laisser agir une composition tinctoriale renfermant au moins un composé de formule (1), (2), ou (3) sur les fibres kératiniques sèches ou humides. On peut utiliser la composition selon l'invention en tant que composition non rincée, c'est-à-dire qu'après application de la composition sur les fibres, on sèche sans rinçage intermédiaire. Dans les autres modes d'application, on laisse agir la composition sur les fibres pendant un temps de pose variant entre 3 et 60 minutes environ, de préférence entre 5 et 45 minutes environ, on rince, éventuellement on lave et on rince à nouveau, puis on sèche.

Des exemples concrets illustrant l'invention vont maintenant être donnés.

### EXEMPLES DE PREPARATION

### EXEMPLE 1 :

### Préparation du 4-(4-diméthylamino-phénvlazo)-N-[3-[1,3,3,3-tétraméthyl-1-[(triméthylsilyl)-oxy]-disiloxanyl]-propyl]-benzènesulfonamide :

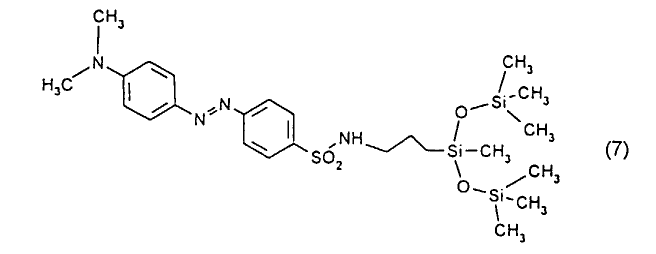

A un mélange au reflux (40°C), d' heptaméthylaminopropyltrisiloxane (3.07g, soit 0,011 mole), de triéthylamine (1,11g, soit 0,011 mole) et de dichlorométhane (10 ml), on a ajouté par portions du chlorure de 4-diméthylamino-4'-azobenzènesulfonyle (3,23g, soit 0,01 mole); on a laissé au reflux pendant 3 heures puis on a refroidi. On a versé le mélange réactionnel dans 30 ml d'eau. On a lavé la phase organique à l'eau puis on l'a séchée sur sulfate de sodium. Après recristallisation dans le méthanol on a obtenu le composé (7) sous la forme d'une poudre orangée.
- Pf : 82 °C
- UV (Ethanol) λₘₐₓ = 432 nm, εₘₐₓ = 37 350.

| -Analyse élémentaire pour C₂₄ H₄₂ N₄ O₄ S Si₃ | | | | | |
|---|---|---|---|---|---|
| théorie | **C**50.85 | **H**7.47 | **N**9.88 | **S**5.66 | **Si**14.86 |
| trouvé | **C**50.53 | **H**7.54 | **N**9.85 | **S**5.51 | **Si**14.90 |

### EXEMPLE 2 :

### Préparation du 4-(4-diméthylamino-phénylazo)-N-[3-[1,3,3,3-tétraméthyl-1-[(triméthylsilyl)-oxy]-disiloxanyl]-propyl]-benzamide :

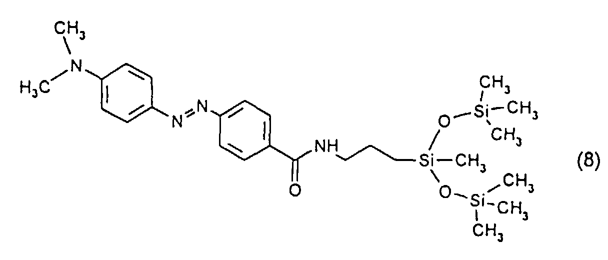

A un mélange au reflux (40°C), d' heptaméthylaminopropyltrisiloxane (6,15g, soit 0,022 mole), de triéthylamine (2,2g, soit 0,022 mole) et de dichlorométhane (20 ml), on a ajouté par portions du chlorure d'acide 4-diméthylamino-azobenzène-4'-carboxylique (3.24 g, soit 0,01 mole); on a laissé au reflux pendant 2 heures puis on a refroidi. On a versé le mélange réactionnel dans 50 ml d'eau. On a lavé la phase organique à l'eau puis on l'a séchée sur sulfate de sodium. On a purifié le produit brut par chromatographie sur colonne (éluant : CH₂Cl₂). On a ainsi obtenu le composé (8) sous la forme d'une poudre orangée.
- Pf : 187 °C
- UV (Ethanol) λₘₐₓ = 428 nm, εₘₐₓ = 33 500.

| -Analyse élémentaire pour C₂₅ H₄₂ N₄ O₃ Si₃ | | | | |
|---|---|---|---|---|
| théorie | **C**56.56 | **H**7.97 | **N**10.55 | **Si**15.87 |
| trouvé | **C**56.32 | **H**7.97 | **N**10.47 | **Si**16.10 |

### EXEMPLE 3 :

### Préparation du 2-(4-méthoxy-2-nitro-phénylazo)-5-(3-triméthylsilanyl-propoxy)-phénol:

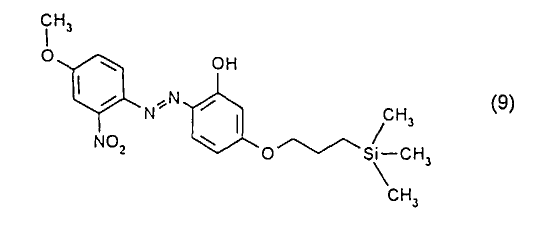

On a porté à 70-75°C un mélange de 4-(4-méthoxy-2-nitro-phénylazo)-benzène-1,3-diol (2.5 g, soit 0.0086 mole) et de carbonate de potassium (1.24 g), dans 30 ml de diméthylformamide. On y a ajouté en 20 minutes du 3-chloropropyltriméthylsilane (1.24 g, soit 0.0086 mole). On a laissé sous agitation à 75°C pendant 6 heures. On a ensuite versé le mélange réactionnel dans 50 ml d'eau puis on a extrait à l'éther diisopropylique. La phase organique a été séchée, concentrée et purifiée par une chromatographie sur colonne (éluant : Heptane/CH₂CI₂ 50:50) pour donner le composé (9) sous la forme d'une poudre rouge.
- Pf : 128-129 °C
- UV (Ethanol) λₘₐₓ = 409 nm, εₘₐₓ = 21 950.

| -Analyse élémentaire pour C₁₉ H₂₅ N₃ O₅ Si | | | | |
|---|---|---|---|---|
| théorie | **C**56.56 | **H**6.24 | **N**10.41 | **Si**6.96 |
| trouvé | **C**56,18 | **H**6,12 | **N**10,46 | **Si**6,69 |

### EXEMPLE 4:

### Préparation du 2,5-bis-[3-[1,3,3,3-tétraméthyl-1-[(triméthylsilyl)-oxy]-disiloxanyl]-propylamino]-benzoquinone :

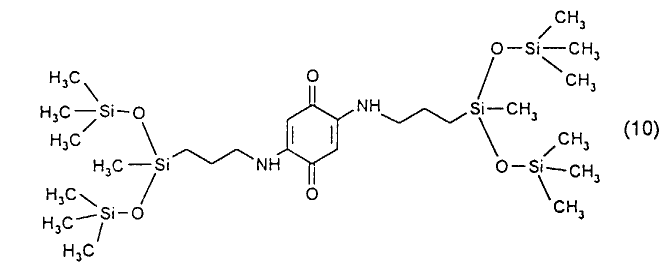

On a porté au reflux pendant 1 heure un mélange de benzoquinone (3.24 g, soit 0.03 mole), d' heptaméthylaminopropyltrisiloxane (5.6 g, soit 0.02 mole), et d'éthanol (40 ml). On a éliminé le solvant sous vide puis repris le résidu dans 50 ml d'heptane. Le mélange a été filtré pour éliminer l'hydroquinone formée et a été purifié par une chromatographie sur colonne (éluant : Heptane/CH₂CI₂ 50:50). On a ainsi obtenu le composé (10) sous la forme d'une poudre rouge.
- Pf : 124 °C
- UV (Ethanol) λₘₐₓ = 341 nm, εₘₐₓ = 30 095.
   λₘₐₓ = 494 nm, εₘₐₓ = 400.

| -Analyse élémentaire pour C₂₆ H₅₈ N₂ O₆ Si₆ | | | | |
|---|---|---|---|---|
| théorie | **C**47.08 | **H**8.81 | **N**4.22 | **Si**25.41 |
| trouvé | **C**46.86 | **H**8.76 | **N**4.23 | **Si**25.80 |

### EXEMPLE 5 :

### Préparation du 2-chloro-3-[3-[1,3,3,3-tétraméthyl-1-[(triméthysilyl)-oxy]-disiloxanyl]-propylamino]-[1,4]-naphtoquinone :

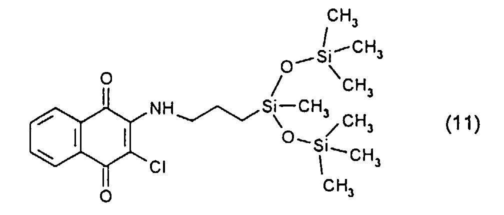

On a porté au reflux 2.27 g, soit 0.01 mole de 2,3-dichloronaphtoquinone dans 30 ml de dichlorométhane. On y a ajouté en 20 minutes de l'heptaméthylaminopropyltrisiloxane (11.2 g, 0.04 mole) et on a laissé au reflux pendant 1 heure. Après concentration du mélange réactionnel, le solide formé a été recristallisé dans le méthanol. On a obtenu le composé (11) sous la forme d'une poudre rouge.
- Pf: 80-81 °C
- UV (Ethanol) λₘₐₓ = 475 nm, εₘₐₓ = 3 850.

| -Analyse élémentaire pour C₂₀ H₃₂ Cl N O₄ Si₃ | | | | | |
|---|---|---|---|---|---|
| théorie | **C**51.09 | **H**6.86 | **C**17.54 | **N**2.98 | **Si**17.92 |
| trouvé | **C**50,72 | **H**6,89 | **C**17,43 | **N**3,12 | **Si**18,28 |

### EXEMPLE 6:

### Préparation du 2-[3-(1,3,3,3-tétraméthyl-1-[(triméthylsilyl)-oxy]-disiloxanyl]-propylamino]-[1,4]-naphtoquinone :

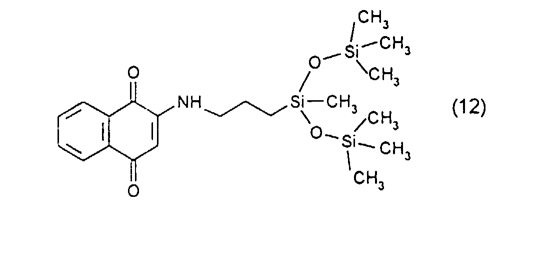

On a porté au reflux pendant 4 heures de la naphtoquinone (4.74 g, soit 0.03 mole) et de l'heptaméthylaminopropyltrisiloxane (5.58 g, soit 0.02 mole) dans de l'éthanol (40 ml). Après concentration de moitié du mélange réactionnel, le solide formé a été isolé. On a obtenu le composé (13) sous la forme d'une poudre orangée.
- Pf: 95-96°C
- UV (Ethanol) λₘₐₓ = 452 nm, εₘₐₓ = 3 850.

### EXEMPLE 7:

Préparation du 1-hydroxy-4-[3-[1,3,3,3-tétraméthyl-1-[(triméthylsilyl)-oxy]-disiloxanyl]-propylamino]-anthraquinone :

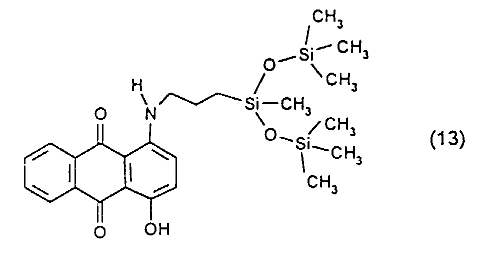

On a porté au reflux pendant 3 heures du leucoquinizarine (4.12 g, soit 0.017 mole) et de l'heptaméthylaminopropyltrisiloxane (11.88 g, soit 0.0425 mole) dans du tétrahydrofuranne (40 ml). Après refroidissement, on a fait barboter un courant d'air dans le mélange réactionnel pendant 2 heures. Après passage chromatographique sur colonne (éluant : Heptane/CH₂Cl₂ 50:50), on a obtenu un mélange des composés (13) et (14). Par cristallisation de ce mélange dans 60 ml de méthanol, on a récupéré le composé (13) sous la forme d'une poudre violette.
- Pf : 71-72 °C
- UV (Ethanol) λₘₐₓ = 557 nm, εₘₐₓ = 13 100.
   λₘₐₓ = 598 nm, εₘₐₓ = 12 400.

| -Analyse élémentaire pour C₂₄ H₃₅ N O₅ Si₃ | | | | |
|---|---|---|---|---|
| théorie | **C**57.45 | **H**7.03 | **N**2.79 | **Si**16.79 |
| trouvé | **C**57.44 | **H**7.04 | **N**2.80 | **Si**16.50 |

### EXEMPLE 8 :

### Préparation du 1,4-di-[3-[1,3,3,3-tétraméthyl-1-[(triméthylsilyl)-oxy]-disiloxanyl]-propylamino]-anthraquinone :

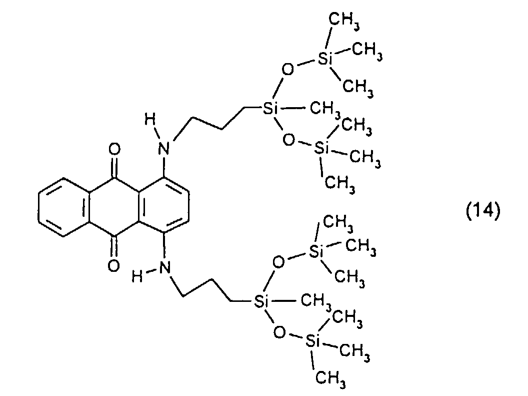

On a porté au reflux pendant 2 heures du leucoquinizarine (2.43 g, soit 0.01 mole) et de l'heptaméthylaminopropyltrisiloxane (8.37 g, soit 0.03 mole) dans un mélange de toluène (50 ml) et de N-méthyl-pyrrolidone (20 ml). Après refroidissement, on a fait barboter un courant d'air dans le mélange réactionnel pendant 2 heures. On a versé le mélange réactionnel dans de l'eau et on a lavé la phase organique à l'eau. Après passage chromatographique sur colonne (éluant : Heptane/CH₂CI₂ 50:50), on a obtenu un mélange des composés (13) et (14). Par cristallisation de ce mélange dans 60 ml de méthanol, on a récupéré le composé (14) sous la forme d'une poudre bleu-foncé.
- Pf: 59-60°C
- UV (Ethanol) λₘₐₓ = 595 nm, εₘₐₓ = 16 950.
   λₘₐₓ = 645 nm, εₘₐₓ = 20 100.

| -Analyse élémentaire pour C₃₄ H₆₂ N₂ O₆ Si₆ | | | | |
|---|---|---|---|---|
| théorie | **C**53.49 | **H**8.19 | **N**3.67 | **Si**22.07 |
| trouvé | **C**53.29 | **H**8.08 | **N**3.65 | **Si**22.10 |

### EXEMPLES DE COMPOSITIONS TINCTORIALES

### EXEMPLE 9 :

On a teint des mèches de cheveux gris naturels à 90% de blancs avec une composition de teinture contenant 5x10⁻² moles du colorant préparé à l'exemple 5, dans une quantité suffisante d'un mélange d'éthanol et d'eau (90/10 en poids) pour porter la composition à 100 g.
Après 30 minutes de traitement, on a rincé les cheveux à l'eau durant 5 minutes, puis on les a séchés.
Les mèches de cheveux ont été teintes en rouge-orangé.

### EXEMPLE 10 :

On a teint des mèches de cheveux gris naturels à 90% de blancs avec une composition de teinture contenant 5x10⁻² moles du colorant préparé à l'exemple 7, dans une quantité suffisante d'un mélange d'éthanol et d'eau (90/10 en poids) pour porter la composition à 100 g.
Après 30 minutes de traitement, on a rincé les cheveux à l'eau durant 5 minutes, puis on les a séchés.
Les mèches de cheveux ont été teintes en rouge-violacé.

## Revendications

1. Utilisation à titre de colorants directs dans des, ou pour la fabrication de, compositions tinctoriales pour fibres kératiniques humaines, de composés de formule (1) ou (2) ou (3) : 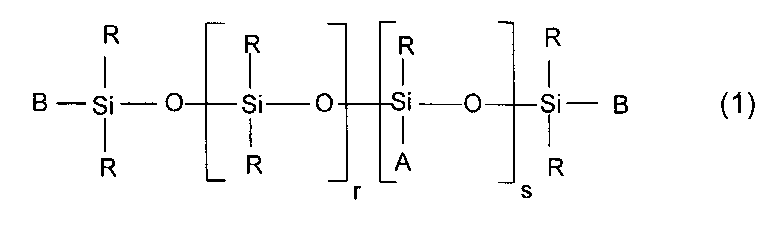 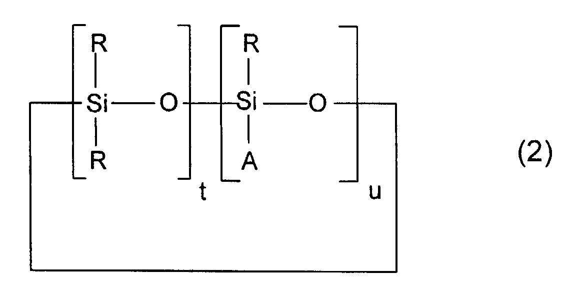 formules (1) ou (2) dans lesquelles :
- **R**, identiques ou différents, sont choisis parmi les radicaux alkyles en C₁-C₁₀, phényle et trifluoro-3,3,3 propyle, au moins 80% en nombre des radicaux R étant méthyle,
- **B, identiques ou différents, sont choisis parmi les radicaux R ci-dessus,**
- **un des deux radicaux B peut désigner le radical A défini ci-après,**
- **r** est un nombre entier compris entre 0 et 50 inclusivement, et **s** est un nombre entier compris entre 0 et 20 inclusivement, avec la condition que si s est nul **alors un des radicaux désigne A,**
- **u** est un nombre entier compris entre 1 et 6 inclusivement, et
- **t** est un nombre entier compris entre 0 et 10 inclusivement, étant entendu que t + u est égal ou supérieur à 3, 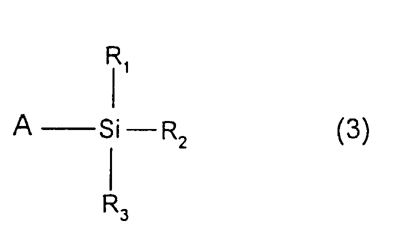 formule (3) dans laquelle:
- **R**_{**1**}, **R**_{**2**}**, R**_{**3**}**,** identiques ou différents, sont choisis parmi les radicaux alkyles et alcényles en C₁-C₈, linéaires ou ramifiés, saturés ou insaturés,
formules (1), (2) et (3) dans lesquelles le symbole **A** désigne un radical identique ou différent de formules (4a) (4b) (4c) (4d) suivantes : 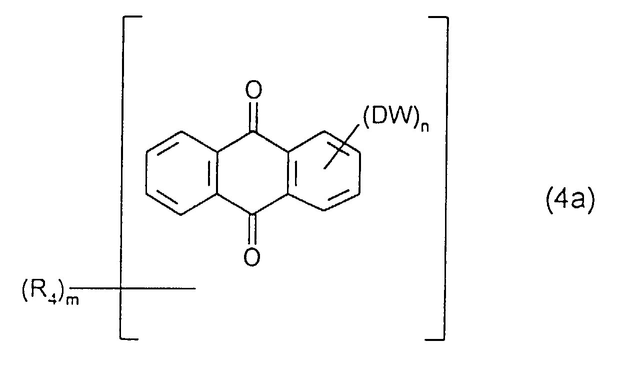 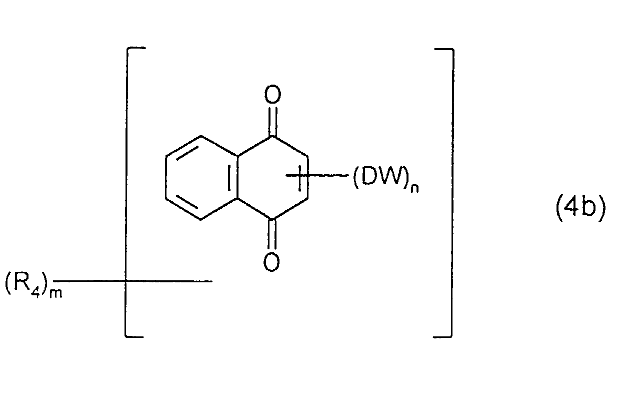 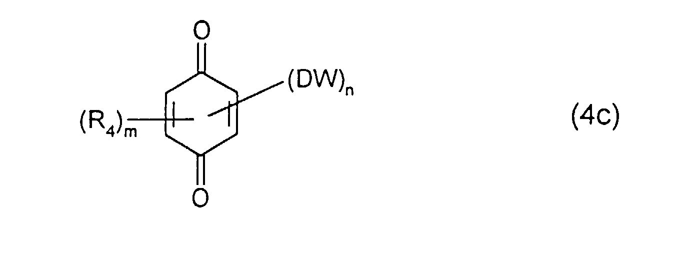 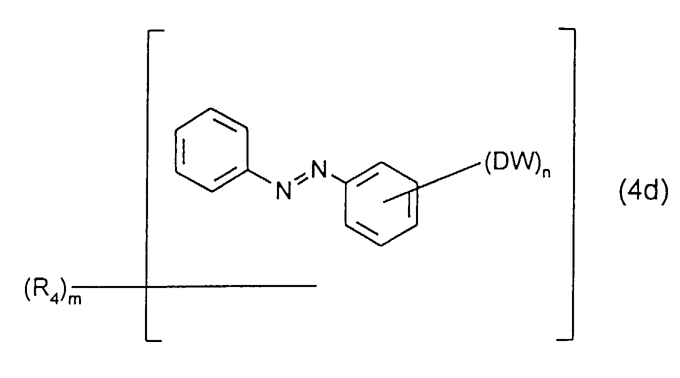 formules (4a) (4b) (4c) (4d) dans lesquelles :
- **R**_{**4**}, identiques ou différents, représentent un radical alkyl, linéaire ou ramifié en C₁-C₆, un radical OH, alcoxy en C₁-C₄, hydroxyalkyl(C₁-C₄), COOH, CONH₂, CN, SO₃H, halogène, NO₂, NR₅R₆ dans lequel **R**_{**5**} et **R**_{**6**}, identiques ou différents, désignent un atome d'hydrogène, un radical alkyl en C₁-C₈, ou hydroxyalkyl(C₁-C₄), ou aminoalkyl(C₁-C₄), ou forment ensemble avec l'atome d'azote auquel ils sont attachés un hétérocycle à Sou 6 chaînons éventuellement interrompu par un atome d'oxygène ou de soufre,
- **m** est un nombre entier compris entre 0 et 2 inclusivement,
- **n** est un nombre entier égal à 1 ou 2,
- **D** est est un radical -SO₂NH-, -CONH-, -O-, ou un radical -NR₇- dans lequel R₇ est H ou CH₃,
- **W** est un radical divalent de formule (5): 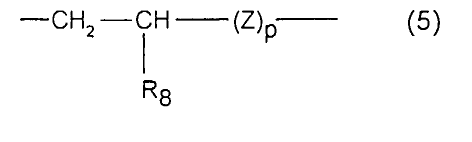 ou de formule (6):
- HC = CH - (Z)ₚ- (6)
formules (5) et (6) dans lesquelles :
- **R**_{**8**} désigne un atome d'hydrogène, un radical hydroxyle, un radical alkyl en C₁-C₈, linéaire ou ramifié, saturé ou insaturé,
- **Z** est un radical alkylène en C₁-C₆, linéaire ou ramifié éventuellement substitué par un radical OH ou un radical alcoxy en C₂-C₈ linéaire ou ramifié, saturé ou insaturé,
- **p** est un nombre entier égal à 0 ou 1.

2. Utilisation des composés de formule (1) ou (2) ou (3) tels que définis à la revendication 1, à titre de colorants directs dans des, ou pour la préparation de, compositions tinctoriales pour cheveux.

3. Utilisation selon les revendications 1 ou 2, caractérisée par le fait que dans la formule (1) ou la formule (2), les radicaux R sont des radicaux méthyle, éthyle, propyle, n-butyle, n-octyle ou éthyl-2 hexyle.

4. Utilisation selon la revendication 3, caractérisée par le fait que les radicaux R sont des radicaux méthyle.

5. Utilisation selon l'une quelconque des revendications précédentes, caractérisée par le fait que dans la formule (1), les radicaux B sont des radicaux méthyle, éthyle, propyle, n-butyle, n-octyle ou éthyl-2 hexyle.

6. Utilisation selon la revendication 5, caractérisée par le fait que les radicaux B sont des radicaux méthyle.

7. Utilisation selon l'une quelconque des revendications précédentes, caractérisée par le fait que dans les composés de formule (1), r est compris entre 0 et 3 inclusivement, et s est compris entre 1 et 3 inclusivement.

8. Utilisation selon l'une quelconque des revendications précédentes, caractérisée par le fait que dans les composés de formule (1), r est nul et s est égal à 1.

9. Utilisation selon l'une quelconque des revendications 1 à 4, caractérisée par le fait que dans les composés de formule (2), t + u est compris entre 3 et 5 inclusivement.

10. Utilisation selon la revendication 1, caractérisée par le fait que dans les composés de formule (3), les radicaux R₁, R₂ et R₃ sont des radicaux méthyle.

11. Utilisation selon l'une quelconque des revendications précédentes, caractérisée par le fait que R₈ est hydrogène, Z est méthylène et p est égal à 1.

12. Utilisation selon l'une quelconque des revendications précédentes, caractérisée par le fait que les composés répondent à l'une des formules suivantes :

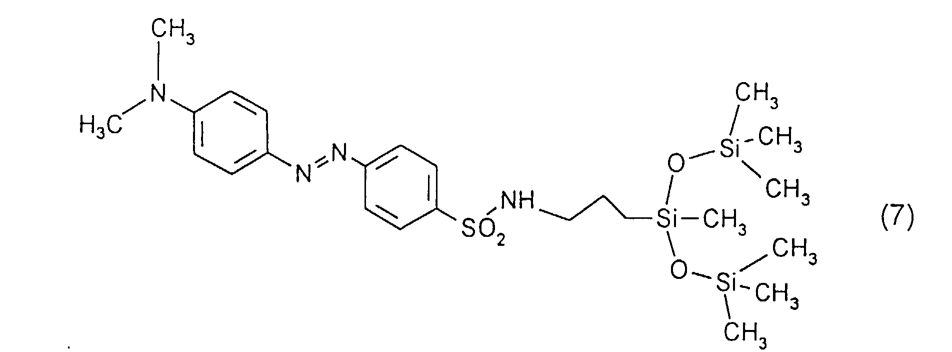

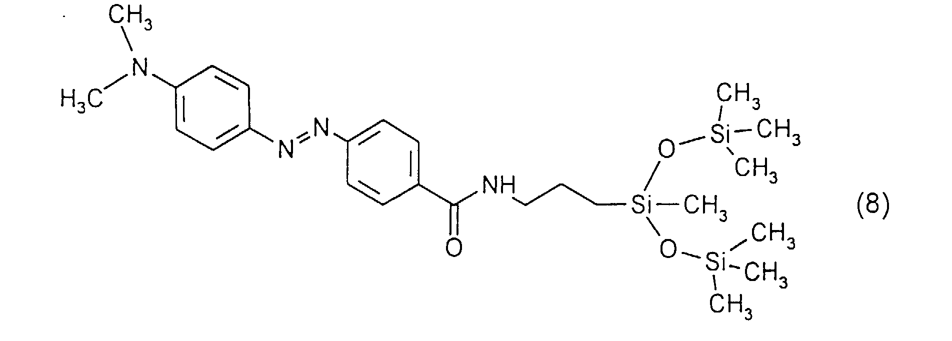

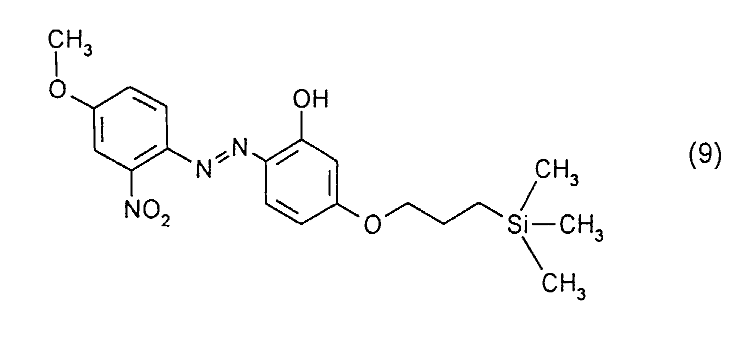

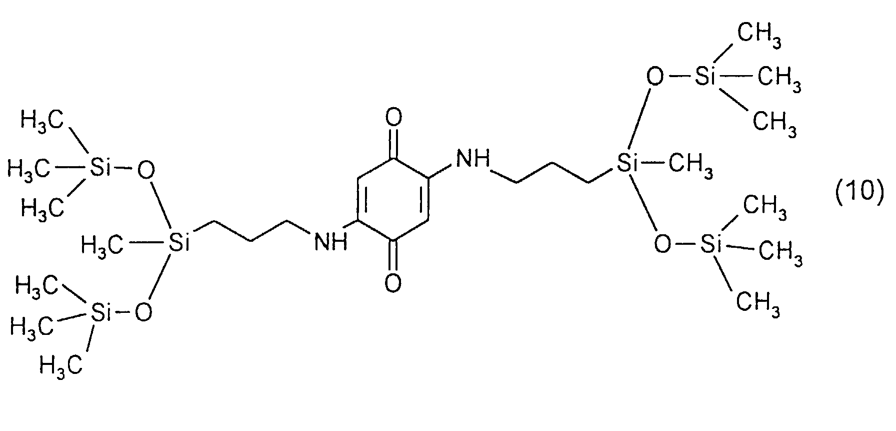

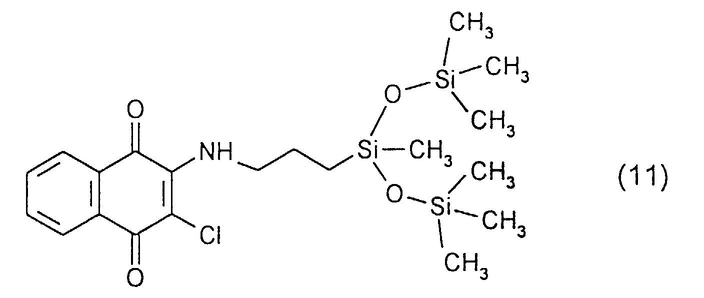

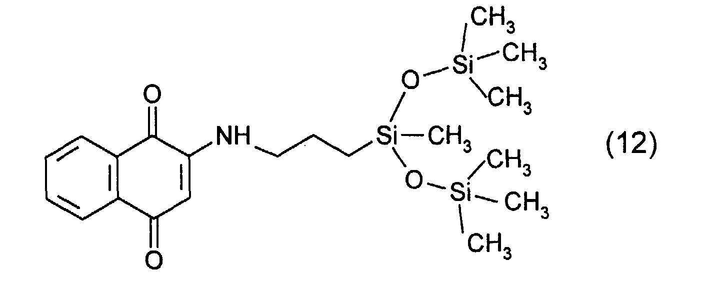

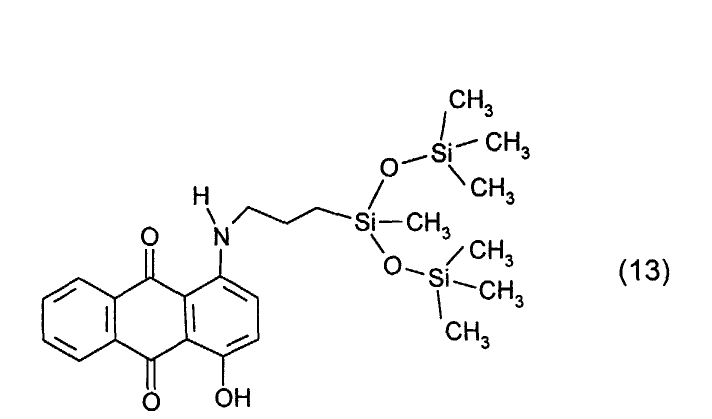

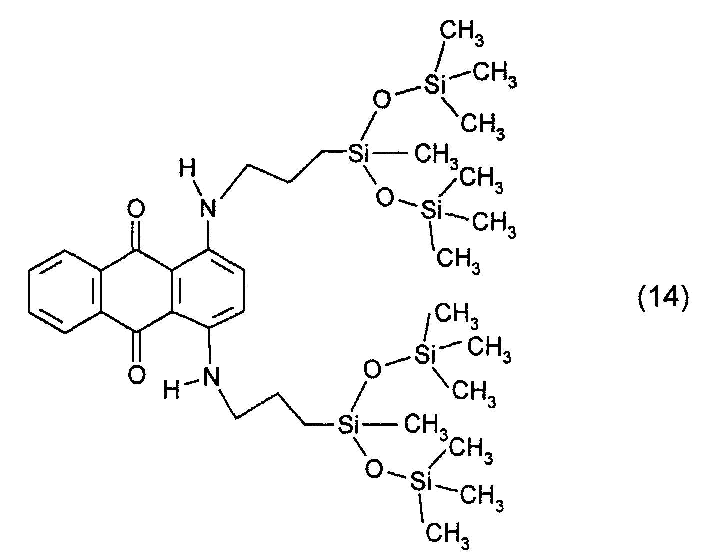

13. Composés de formule (1) selon la revendication 1,
**sous réserve que, lorsque :**
l**es radicaux B désignent le radical CH**_{**3**}**, s soit égal à 1, et r désigne un nombre entier compris entre 0 et 10 inclusivement.**

14. Composés de formule (2) selon la revendication 1.

15. Composés de formule (3) selon la revendication 1,
sous réserve qu'ils soient différents des composés de formule (3) telle que définie ci-dessus pour lesquels A est un groupe de formule (4d), R₈ désigne l'hydrogène, R₁, R₂ et R₃ désignent des groupes alkyle et D un groupe NR₇.

16. Composés de formules (7) ; (8) ; (9) ; (10) ; (11) ; (12) ; (13) ; (14) , tels que définis selon la revendication 12.

17. Composition tinctoriale pour fibres kératiniques humaines et notamment les cheveux caractérisée par le fait qu'elle comprend, dans un milieu approprié pour la teinture, au moins un des composés de formule (1) ou (2) ou (3) définis à l'une quelconque des revendications 1-12.

18. Composition selon la revendication 17, caractérisée par le fait qu'il s'agit de teinture directe.

19. Composition selon les revendications 17 et 18, caractérisée par le fait qu'elle a un pH compris entre 4 et 11.

20. Composition selon les revendications 17 à 19, caractérisée par le fait que les composés de formule (1) ou (2) ou (3) sont présents dans une concentration allant de 0,01 à10% en poids par rapport au poids total de la composition.

21. Composition selon les revendications 17 à 20, caractérisée par le fait que le milieu approprié pour la teinture est constitué par de l'eau et/ou des solvants organiques tels que des alcools, des glycols, des éthers de glycols, dans des proportions comprises entre 0,5 et 20% en poids par rapport au poids total de la composition.

22. Procédé de teinture des fibres kératiniques humaines et notamment des cheveux, par coloration directe, caractérisé par le fait qu'on applique une composition tinctoriale renfermant au moins un composé de formule (1) ou (2) ou (3) tel que défini à l'une quelconque des revendications 1-12, sur les fibres kératiniques, sèches ou humides, et qu'après avoir éventuellement laissé agir la composition sur les fibres pendant un temps de pose allant de 3 à 60 minutes, on sèche ces fibres, après un rinçage éventuel.

## Patentansprüche

1. Verwendung von Verbindungen der Formel (1) oder (2) oder (3) als Direktfarbstoffe in Färbemittelzusammensetzungen für menschliche Keratinfasern oder zur Herstellung dieser Zusammensetzungen: 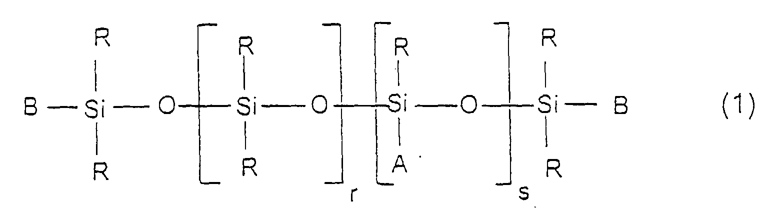 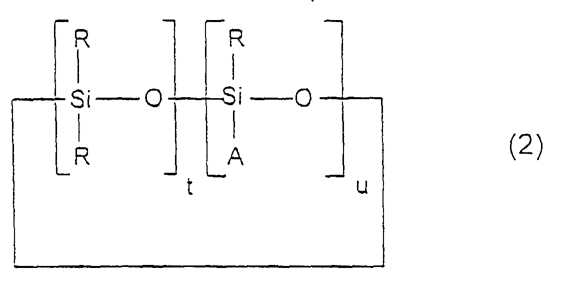 wobei in den Formeln (1) oder (2)
- die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Gruppen R Methyl bedeuten,
- die Gruppen B, die identisch oder voneinander verschieden sind, unter den oben genannten Gruppen R ausgewählt sind,
- eine der beiden Gruppen B die nachstehend definierte Gruppe A bedeuten kann,
- r 0 oder eine ganze Zahl im Bereich von 1 bis 50 und s 0 oder eine ganze Zahl im Bereich von 1 bis 20 bedeutet, mit der Maßgabe, daß eine der Gruppen B A bedeutet, wenn s 0 ist,
- u eine ganze Zahl im Bereich von 1 bis 6 bedeutet, und
- t 0 oder eine ganze Zahl im Bereich von 1 bis 10 bedeutet, mit der Maßgabe, daß t + u ≥ 3 ist,
und 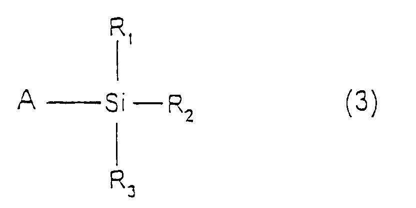 wobei in der Formel (3)
- die Gruppen R₁, R₂ und R₃, die identisch oder voneinander verschieden sind, unter den geradkettigen oder verzweigten, gesättigten oder ungesättigten Alkyl- und Alkenylgruppen mit 1 bis 8 Kohlenstoffatomen ausgewählt sind,
wobei in den Formeln (1), (2) und (3) die Gruppen A, die identisch oder voneinander verschieden sind, die folgenden Formeln (4a), (4b), (4c) und (4d) bedeuten: 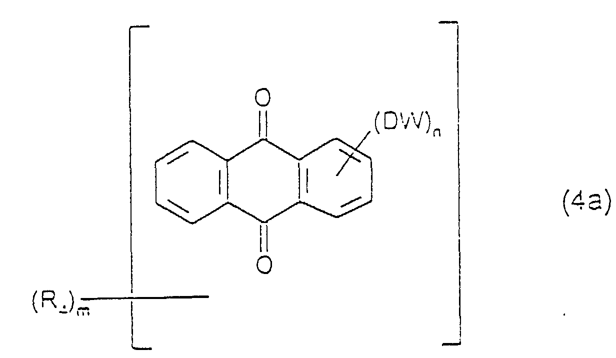 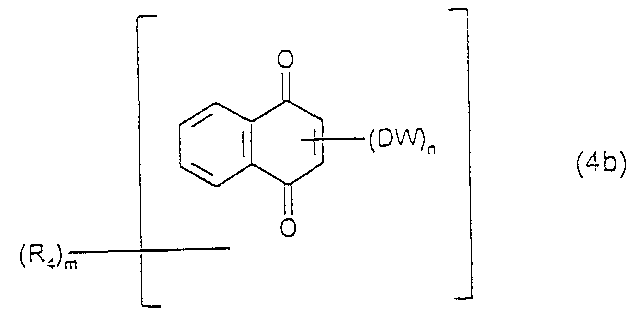 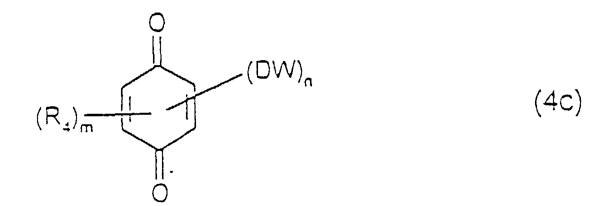 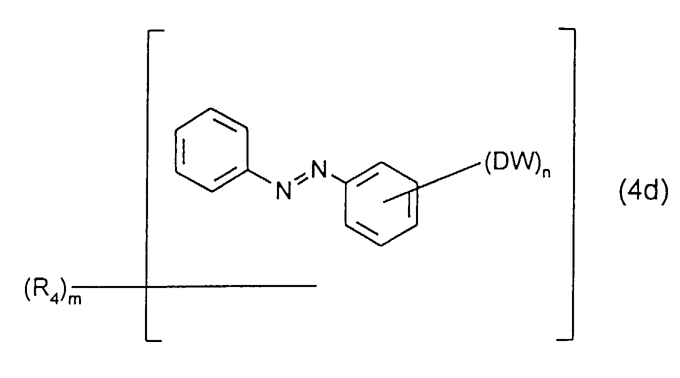 wobei in den Formeln (4a), (4b), (4c) und (4d):
- die Gruppen R₄, die identisch oder voneinander verschieden sind, eine geradkettige oder verzweigte C₁₋₆-Alkylgruppe, OH, C₁₋₄-Alkoxy, C₁₋₄-Hydroxyalkyl, COOH, CONH₂, CN, SO₃H, Halogen, NO₂ oder NR₅R₆bedeuten, worin die Gruppen R₅ und R₆, die identisch oder voneinander verschieden sind, Wasserstoff, C₁₋₈-Alkyl, C₁₋₄-Hydroxyalkyl oder C₁₋₄-Aminoalkyl bedeuten oder gemeinsam mit dem Stickstoffatom, an das sie gebunden sind, einen 5- oder 6-gliedrigen Heterocyclus bilden, der ggf. mit einem Sauerstoff- oder Schwefelatom unterbrochen ist,
- m 0 oder eine ganze Zahl 1 oder 2 bedeutet,
- n eine ganze Zahl 1 oder 2 ist,
- D eine Gruppe -SO₂NH-, -CONH-, -O- oder eine Gruppe -NR₇- ist, worin R₇ H oder CH₃ bedeutet,
- W eine zweiwertige Gruppe der Formel (5) 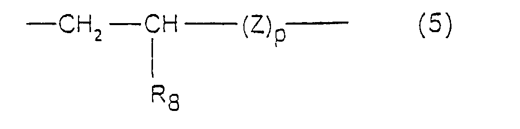 oder der Formel (6),
-HC=CH(Z)ₚ- (6)
bedeutet, wobei in den Formeln (5) und (6) bedeuten:
- R₈ Wasserstoff, Hydroxy oder eine geradkettige oder verzweigte, gesättigte oder ungesättigte C₁₋₈-Alkylgruppe,
- Z eine geradkettige oder verzweigte C₁₋₆-Alkylengruppe, die ggf. mit einer OH-Gruppe oder einer geradkettigen oder verzweigten, gesättigten oder ungesättigten C₂₋₈-Alkoxygruppe substituiert ist, und
- p 0 oder 1.

2. Verwendung der Verbindungen der Formel (1) oder (2) oder (3) nach Anspruch 1 als Direktfarbstoffe in Färbemittelzusammensetzungen für das Haar oder zur Herstellung dieser Zusammensetzungen.

3. Verwendung nach Anspruch 1 oder 2, dadurch gekennzeichnet, daß in der Formel (1) oder in der Formel (2) die Gruppen R die Gruppen Methyl, Ethyl, Propyl, n-Butyl, n-Octyl oder 2-Ethylhexyl bedeuten.

4. Verwendung nach Anspruch 3, dadurch gekennzeichnet, daß die Gruppen R Methylgruppen sind.

5. Verwendung nach einem der vorhergehenden Ansprüche, dadurch gekennzeichnet, daß in der Formel (1) die Gruppen B die Gruppen Methyl, Ethyl, Propyl, n-Butyl, n-Octyl oder 2-Ethylhexyl bedeuten.

6. Verwendung nach Anspruch 5, dadurch gekennzeichnet, daß die Gruppen B Methylgruppen sind.

7. Verwendung nach einem der vorhergehenden Ansprüche, dadurch gekennzeichnet, daß in den Verbindungen der Formel (1) r im Bereich von 0 bis 3 und s im Bereich von 1 bis 3 liegt.

8. Verwendung nach einem der vorhergehenden Ansprüche, dadurch gekennzeichnet, daß in den Verbindungen der Formel (1) r 0 und s 1 bedeutet.

9. Verwendung nach einem der Ansprüche 1 bis 4, dadurch gekennzeichnet, daß in den Verbindungen der Formel (2) t + u im Bereich von 3 bis 5 liegt.

10. Verwendung nach Anspruch 1, dadurch gekennzeichnet, daß in den Verbindungen der Formel (3) die Gruppen R₁, R₂ und R₃ Methylgruppen sind.

11. Verwendung nach einem der vorhergehenden Ansprüche, dadurch gekennzeichnet, daß R₈ Wasserstoff, Z Methylen und p 1 bedeutet.

12. Verwendung nach einem der vorhergehenden Ansprüche, dadurch gekennzeichnet, daß die Verbindungen einer der folgenden Formeln entsprechen

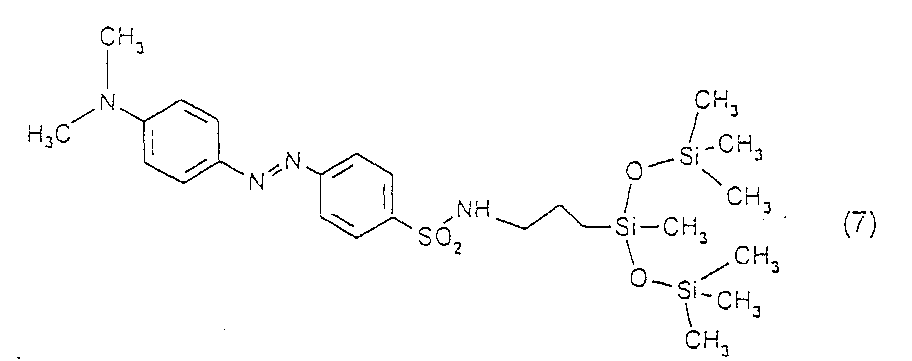

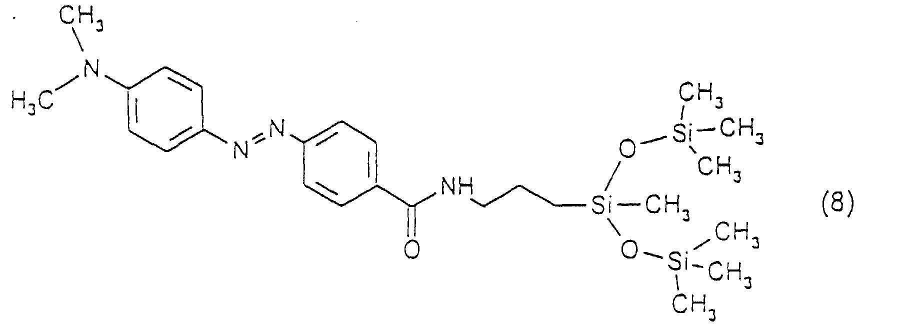

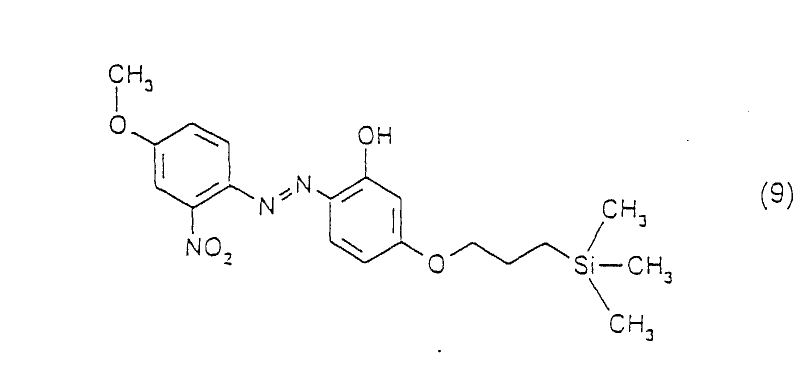

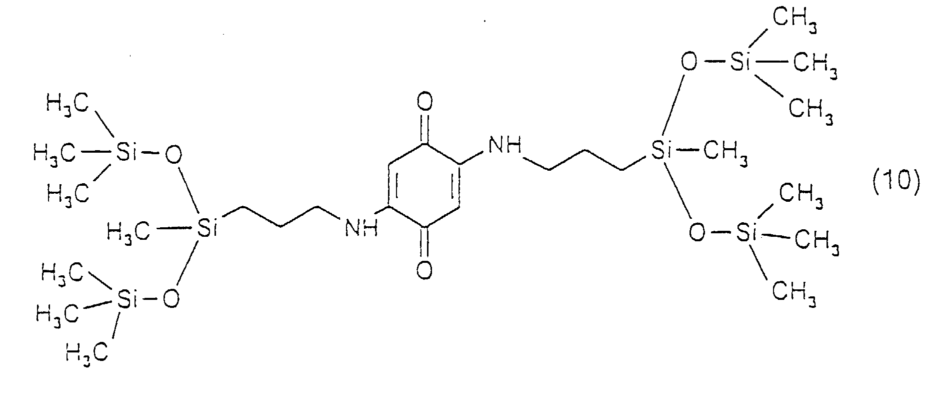

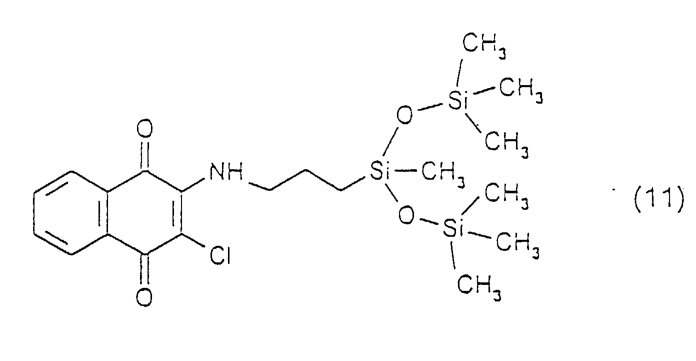

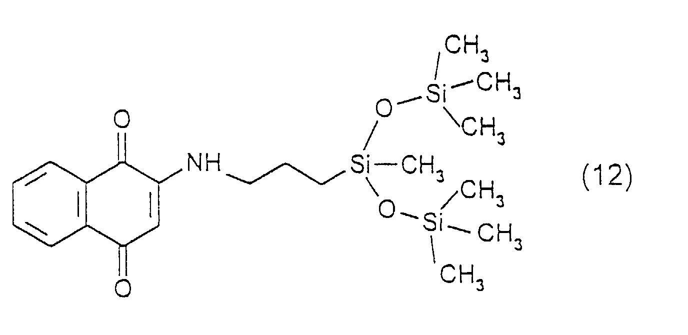

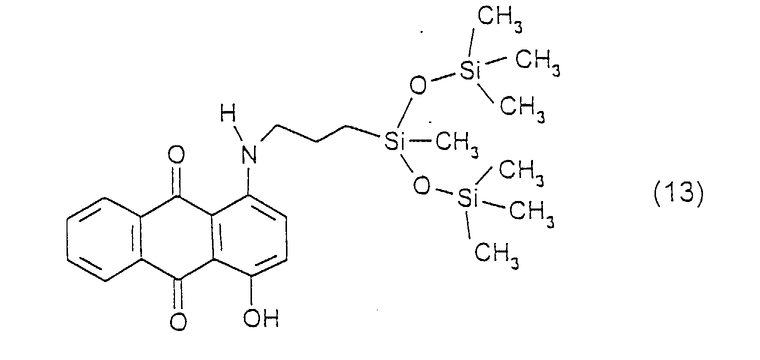

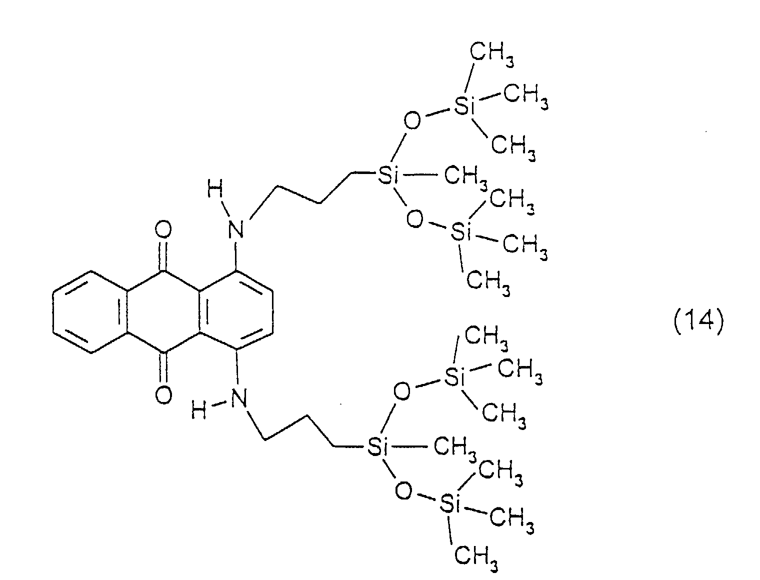

13. Verbindungen der Formel (1) nach Anspruch 1, mit der Maßgabe, daß bedeuten:
die Gruppen B die Gruppe CH₃, s 1 und r 0 oder eine ganze Zahl im Bereich von 1 bis 10.

14. Verbindungen der Formel (2) nach Anspruch 1.

15. Verbindungen der Formel (3) nach Anspruch 1, mit der Maßgabe, daß sie von den Verbindungen der oben definierten Formel (3) verschieden sind, worin A eine Gruppe (4d), R₈ Wasserstoff, R₁, R₂ und R₃ Alkylgruppen und D eine Gruppe NR₇ bedeuten.

16. Verbindungen der Formeln (7), (8), (9), (10), (11), (12), (13), (14) nach Anspruch 12.

17. Färbemittelzusammensetzung für menschliche Keratinfasern und insbesondere das Haar, dadurch gekennzeichnet, daß sie in einem zum Färben geeigneten Medium mindestens eine Verbindung der Formel (1) oder (2) oder (3) nach einem der Ansprüche 1 bis 12 enthält.

18. Zusammensetzung nach Anspruch 17, dadurch gekennzeichnet, daß es sich um eine Direktfärbung handelt.

19. Zusammensetzung nach den Ansprüchen 17 und 18, dadurch gekennzeichnet, daß sie einen pH-Wert im Bereich von 4 bis 11 aufweist.

20. Zusammensetzung nach den Ansprüchen 17 bis 19, dadurch gekennzeichnet, daß die Verbindungen der Formel (1) oder (2) oder (3) in einer Konzentration im Bereich von 0,01 bis 10 Gew.-%, bezogen auf das Gesamtgewicht der Zusammensetzung, vorliegen.

21. Zusammensetzung nach den Ansprüchen 17 bis 20, dadurch gekennzeichnet, daß das zum Färben geeignete Medium aus Wasser und/oder organischen Lösungsmitteln, wie Alkoholen, Glykolen und Glykolethern in Mengenanteilen im Bereich von 0,5 bis 20 Gew.-%, bezogen auf das Gesamtgewicht der Zusammensetzung, besteht.

22. Verfahren zum Färben von menschlichen Keratinfasern insbesondere dem Haar durch direkte Färbung, dadurch gekennzeichnet, daß auf die trockenen oder feuchten Keratinfasern eine Färbemittelzusammensetzung aufgetragen wird, die mindestens eine Verbindung der Formel (1) oder (2) oder (3) nach einem der Ansprüche 1 bis 12 enthält, die Zusammensetzung ggf. während einer Einwirkzeit von 3 bis 60 min auf die Fasern einwirken gelassen wird, und die Fasern getrocknet werden, nachdem sie ggf. gespült wurden.

## Claims

1. Use, as direct dyes, in, or for the manufacture of, dye compositions for human keratin fibres, of compounds of formula (1), (2) or (3) : 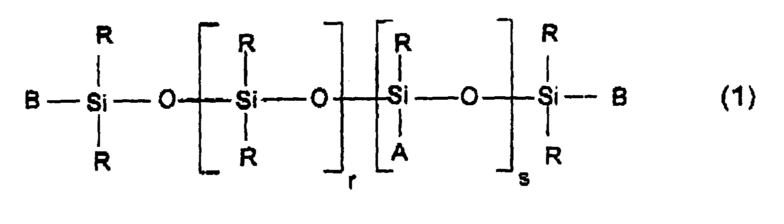 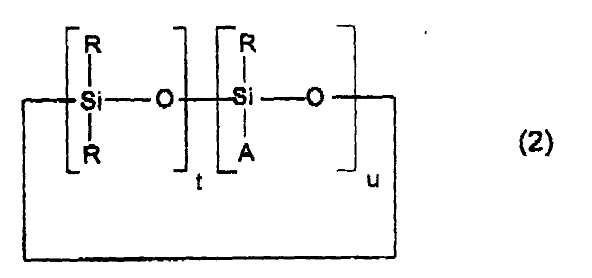 in which formulae (1) and (2) :
- R, which may be identical or different, are chosen from C₁-C₁₀ alkyl, phenyl and 3,3,3-trifluoropropyl radicals, at least 80%, in numerical terms, of the radicals R being methyl,
- B, which may be identical or different, are chosen from the above radicals R,
- one of the two radicals B may denote the radical A defined below,
- r is an integer between 0 and 50 inclusive, and s is an integer between 0 and 20 inclusive, with the condition that if s is zero then one of the radicals denotes A,
- u is an integer between 1 and 6 inclusive, and
- t is an integer between 0 and 10 inclusive, it being understood that t + u is equal to or greater than 3, 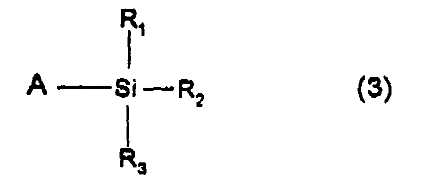 in which formula (3):
- R₁, R₂ and R₃, which may be identical or different, are chosen from linear or branched, saturated or unsaturated C₁-C₈ alkyl and alkenyl radicals,
in which formulae (1), (2) and (3) the symbol A denotes a radical, which may be identical or different, of formula (4a), (4b), (4c) or (4d) below: 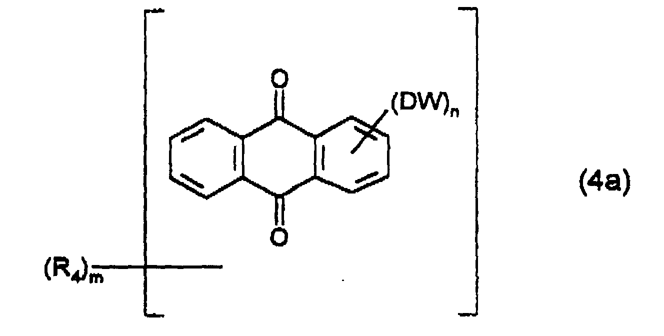 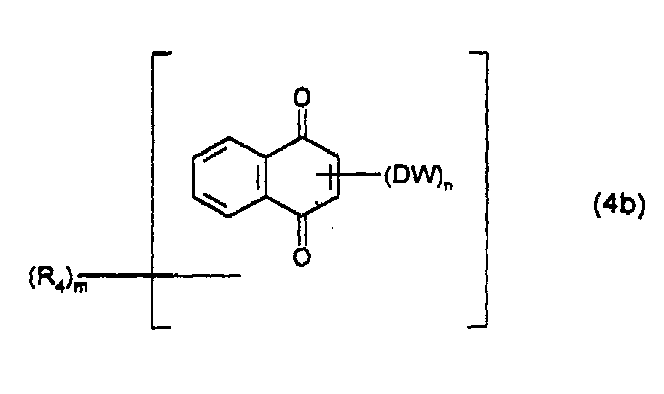 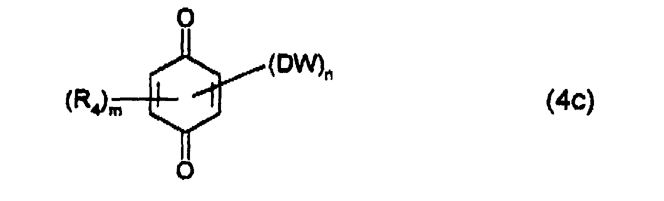 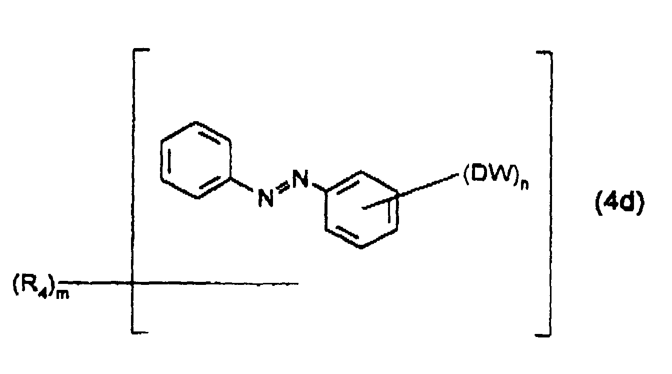 in which formulae (4a), (4b), (4c) and (4d):
- R₄, which may be identical or different, represent a linear or branched C₁-C₆ alkyl radical, an OH, C₁-C₄ alkoxy, hydroxy(C₁-C₄)alkyl, COOH, CONH₂, CN, SO₃H, halogen or NO₂ radical, a radical NR₅R₆ in which R₅ and R₆, which may be identical or different, denote a hydrogen atom, a C₁-C₈ alkyl or hydroxy(C₁-C₄)alkyl or amino(C₁-C₄)alkyl radical, or form, together with the nitrogen atom to which they are attached, a 5- or 6-membered heterocycle which may be interrupted by an oxygen or sulphur atom,
- m is an integer between 0 and 2 inclusive,
- n is an integer equal to 1 or 2,
- D is an -SO₂NH-, -CONH- or -O- radical or a radical -NR₇- in which R₇ is H or CH₃,
- W is a divalent radical of formula (5): 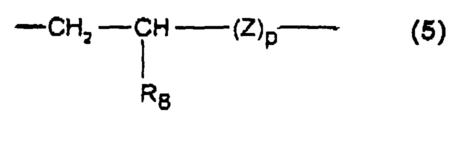 or of formula (6):
- HC=CH-(Z)ₚ- (6)
in which formulae (5) and (6):
- R₈ denotes a hydrogen atom, a hydroxyl radical or a linear or branched, saturated or unsaturated C₁-C₈ alkyl radical,
- Z is a linear or branched C₁-C₆ alkylene radical optionally substituted with an OH radical or a linear or branched, saturated or unsaturated C₂-C₈ alkoxy radical,
- p is an integer equal to 0 or 1.

2. Use of the compounds of formula (1), (2) or (3) as defined in Claim 1, as direct dyes, in, or for the preparation of, dye compositions for hair.

3. Use according to Claim 1 or 2, characterized in that, in formula (1) or formula (2), the radicals R are methyl, ethyl, propyl, n-butyl, n-octyl or 2-ethylhexyl radicals.

4. Use according to Claim 3, characterized in that the radicals R are methyl radicals.

5. Use according to any one of the preceding claims, characterized in that, in formula (1), the radicals B are methyl, ethyl, propyl, n-butyl, n-octyl or 2-ethylhexyl radicals.

6. Use according to Claim 5, characterized in that the radicals B are methyl radicals.

7. Use according to any one of the preceding claims, characterized in that, in the compounds of formula (1), r is between 0 and 3 inclusive, and s is between 1 and 3 inclusive.

8. Use according to any one of the preceding claims, characterized in that, in the compounds of formula (1), r is zero and s is equal to 1.

9. Use according to any one of Claims 1 to 4, characterized in that, in the compounds of formula (2), t + u is between 3 and 5 inclusive.

10. Use according to Claim 1, characterized in that, in the compounds of formula (3), the radicals R₁, R₂ and R₃ are methyl radicals.

11. Use according to any one of the preceding claims, characterized in that R₈ is hydrogen, Z is methylene and p is equal to 1.

12. Use according to any one of the preceding claims, characterized in that the compounds correspond to one of the following formulae:

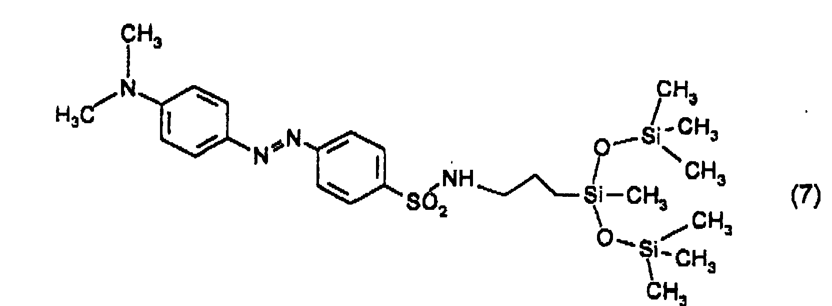

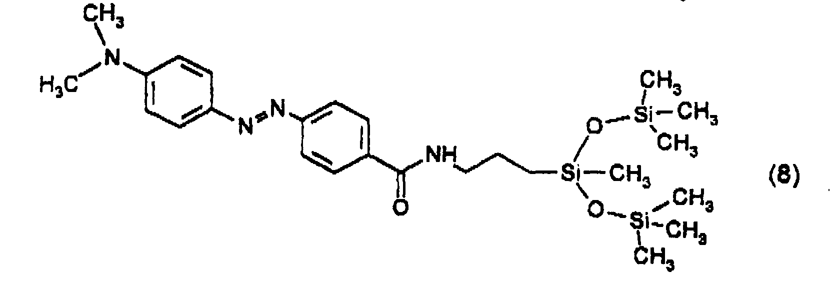

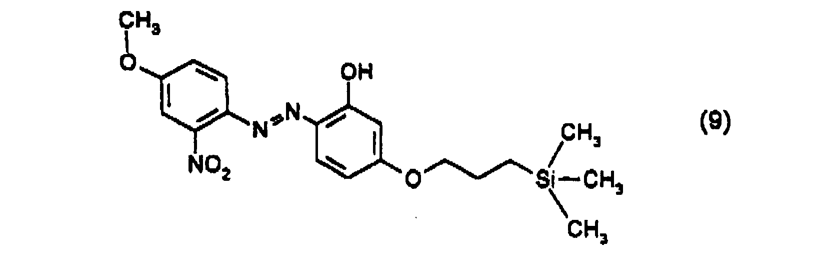

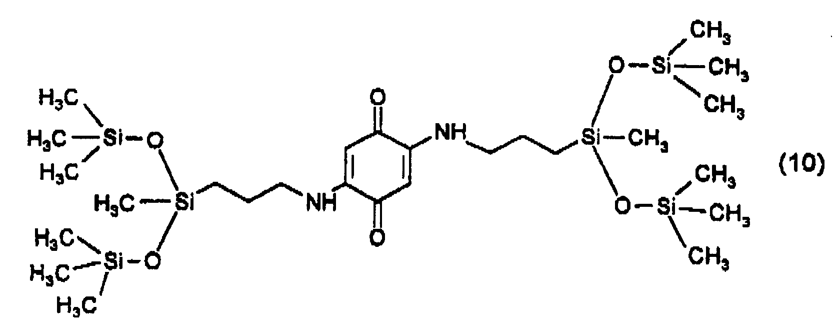

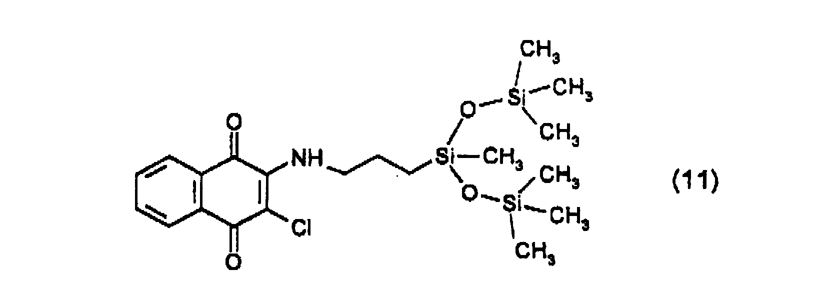

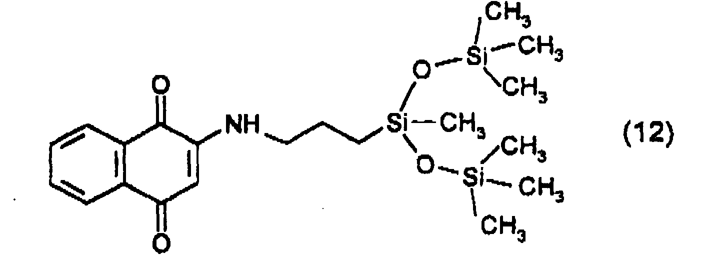

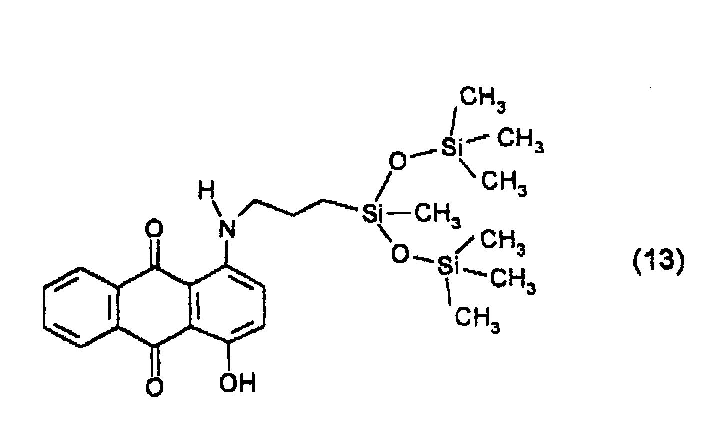

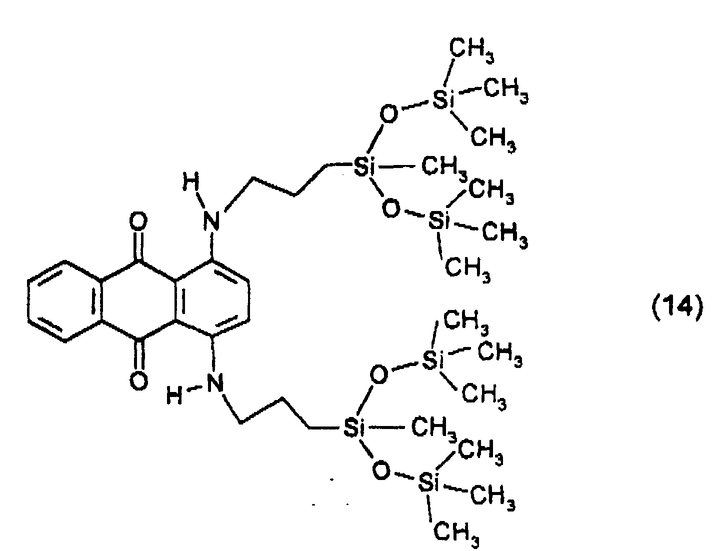

13. Compounds of formula (1) according to Claim 1, with the proviso that, when:
the radicals B denote a CH₃ radical, s is equal to 1, and r denotes an integer between 0 and 10 inclusive.

14. Compounds of formula (2) according to Claim 1.

15. Compounds of formula (3) according to Claim 1, with the proviso that they are different from the compounds of formula (3) as defined above for which A is a group of formula (4d), R₈ denotes hydrogen, R₁, R₂ and R₃ denote alkyl groups and D denotes a group NR₇.

16. Compounds of formulae (7); (8); (9); (10); (11); (12); (13); (14), as defined according to Claim 12.

17. Dye composition for human keratin fibres and in particular the hair, characterized in that it comprises, in a medium which is suitable for dyeing, at least one of the compounds of formula (1), (2) or (3) defined in any one of Claims 1-12.

18. Composition according to Claim 17, characterized in that it is a direct dye composition.

19. Composition according to Claims 17 and 18, characterized in that it has a pH of between 4 and 11.

20. Composition according to Claims 17 to 19, characterized in that the compounds of formula (1), (2) or (3) are present in a concentration ranging from 0.01 to 10% by weight relative to the total weight of the composition.

21. Composition according to Claims 17 to 20, characterized in that the medium which is suitable for dyeing consists of water and/or organic solvents such as alcohols, glycols or glycol ethers, in proportions of between 0.5 and 20% by weight relative to the total weight of the composition.

22. Process for dyeing human keratin fibres and in particular the hair, by direct dyeing, characterized in that a dye composition containing at least one compound of formula (1), (2) or (3) as defined in any one of Claims 1-12 is applied to wet or dry keratin fibres and, after the composition has optionally been left on the fibres for an exposure time ranging from 3 to 60 minutes, these fibres are dried, after an optional rinsing operation.
